# EUROPEAN PATENT APPLICATION

(11) **EP 1 489 179 A1**
(43) Date of publication of application: **22.12.2004**
(21) Application number: 03715524.9
(22) Date of filing: 27.03.2003
(51) Int. Cl.: C12N 15/12, C12N 1/15, C12N 1/19, C12N 1/21, C12N 5/10, C07K 14/485, A01K 67/027, A61K 35/76, A61K 38/00, A61K 48/00, A61P 17/02, A61P 43/00

(54) **ANTIOPOIETIN-RELATED GROWTH FACTOR**

(30) Priority: 28.03.2002 JP 2002093128; 18.12.2002 JP 2002366378
(71) Applicant: YAMANOUCHI PHARMACEUTICAL CO. LTD., Tokyo 103-8411 (JP)
(72) Inventor: YASUNAGA, K., Yamanouchi Pharmaceutical Co., Ltd., Tsukuba-shi, Ibaraki 305-0841 (JP); YAMAJI, N., Yamanouchi Pharmaceutical Co., Ltd., Tsukuba-shi, Ibaraki 305-0841 (JP); MATSUMOTO, Shunichiro, Kashiwa-shi, Chiba 277-0872 (JP); SUDA, Toshio, Chiyoda-ku, Tokyo 102-0093 (JP); OIKE, Yuichi, Kumamoto-shi, Kumamoto 862-0971 (JP)
(74) Representative: HOFFMANN - EITLE
(86) International application number: PCT/JP2003/003847
(87) International publication number: WO 2003/083114

(57) **Abstract**

The present invention provides a mouse ortholog gene (AGF) of known human gene, NL8/NEW. AGF has proliferative action for epidermal cells, is therefore useful as a wound healing agent or a tissue regenerative agent. The present invention demonstrates that a transgenic mouse manipulated to express AGF in the epidermal cells can be used as an animal model for psoriasis.

SEQUENCE LISTING

<110> YAMANOUCHI PHARMACEUTICAL CO., LTD.

<120> Angiopoietin-related growth factors

<130> Y1-X0202P

<140>
<141>

<150> JP 2002-093128
<151> 2002-03-28

<150> JP 2002-366378
<151> 2002-12-18

<160> 19

<170> PatentIn Ver. 2.1

<210> 1
<211> 1413
<212> DNA
<213> Homo sapiens

<220>

<221> CDS

<222> (1)..(1410)

<220>

<221> mat_peptide

<222> (61)..(1410)

<400> 1

atg ggg aag ccc tgg ctg cgt gcg cta cag ctg ctg ctc ctg ctg ggc 48

Met Gly Lys Pro Trp Leu Arg Ala Leu Gln Leu Leu Leu Leu Leu Gly

-20 -15 -10 -5

gcg tcg tgg gcg cgg gcg ggc gcc ccg cgc tgc acc tac acc ttc gtg 96

Ala Ser Trp Ala Arg Ala Gly Ala Pro Arg Cys Thr Tyr Thr Phe Val

-1 1 5 10

ctg ccc ccg cag aag ttc acg ggc gct gtg tgc tgg agc ggc ccc gca 144

Leu Pro Pro Gln Lys Phe Thr Gly Ala Val Cys Trp Ser Gly Pro Ala

15 20 25

tcc acg cgg gcg acg ccc gag gcc gcc aac gcc agc gag ctg gcg gcg 192

Ser Thr Arg Ala Thr Pro Glu Ala Ala Asn Ala Ser Glu Leu Ala Ala

30 35 40

ctg cgc atg cgc gtc ggc cgc cac gag gag ctg tta cgc gag ctg cag 240

Leu Arg Met Arg Val Gly Arg His Glu Glu Leu Leu Arg Glu Leu Gln
45 50 55 60

agg ctg gcg gcg gcc gac ggc gcc gtg gcc ggc gag gtg cgc gcg ctg 288
Arg Leu Ala Ala Ala Asp Gly Ala Val Ala Gly Glu Val Arg Ala Leu
65 70 75

cgc aag gag agc cgc ggc ctg agc gcg cgc ctg ggc cag ttg cgc gcg 336
Arg Lys Glu Ser Arg Gly Leu Ser Ala Arg Leu Gly Gln Leu Arg Ala
80 85 90

cag ctg cag cac gag gcg ggg ccc ggg gcg ggc ccg ggg gcg gat ctg 384
Gln Leu Gln His Glu Ala Gly Pro Gly Ala Gly Pro Gly Ala Asp Leu
95 100 105

ggg gcg gag cct gcc gcg gcg ctg gcg ctg ctc ggg gag cgc gtg ctc 432
Gly Ala Glu Pro Ala Ala Ala Leu Ala Leu Leu Gly Glu Arg Val Leu
110 115 120

aac gcg tcc gcc gag gct cag cgc gca gcc gcc cgg ttc cac cag ctg 480
Asn Ala Ser Ala Glu Ala Gln Arg Ala Ala Ala Arg Phe His Gln Leu
125 130 135 140

gac gtc aag ttc cgc gag ctg gcg cag ctc gtc acc cag cag agc agt 528
Asp Val Lys Phe Arg Glu Leu Ala Gln Leu Val Thr Gln Gln Ser Ser
145 150 155

ctc atc gcc cgc ctg gag cgc ctg tgc ccg gga ggc gcg ggc ggg cag 576
Leu Ile Ala Arg Leu Glu Arg Leu Cys Pro Gly Gly Ala Gly Gly Gln
160 165 170

cag cag gtc ctg ccg cca ccc cca ctg gtg cct gtg gtt ccg gtc cgt 624
Gln Gln Val Leu Pro Pro Pro Pro Leu Val Pro Val Val Pro Val Arg
175 180 185

ctt gtg ggt agc acc agt gac acc agt agg atg ctg gac cca gcc cca 672
Leu Val Gly Ser Thr Ser Asp Thr Ser Arg Met Leu Asp Pro Ala Pro
190 195 200

gag ccc cag aga gac cag acc cag aga cag cag gag ccc atg gct tct 720
Glu Pro Gln Arg Asp Gln Thr Gln Arg Gln Gln Glu Pro Met Ala Ser
205 210 215 220

ccc atg cct gca ggt cac cct gcg gtc ccc acc aag cct gtg ggc ccg 768
Pro Met Pro Ala Gly His Pro Ala Val Pro Thr Lys Pro Val Gly Pro
225 230 235

tgg cag gat tgt gca gag gcc cgc cag gca ggc cat gaa cag agt gga 816
Trp Gln Asp Cys Ala Glu Ala Arg Gln Ala Gly His Glu Gln Ser Gly
240 245 250

gtg tat gaa ctg cga gtg ggc cgt cac gta gtg tca gta tgg tgt gag 864

Val Tyr Glu Leu Arg Val Gly Arg His Val Val Ser Val Trp Cys Glu
255 260 265

cag caa ctg gag ggt gga ggc tgg act gtg atc cag cgg agg caa gat 912
Gln Gln Leu Glu Gly Gly Gly Trp Thr Val Ile Gln Arg Arg Gln Asp
270 275 280

ggt tca gtc aac ttc ttc act acc tgg cag cac tat aag gcg ggc ttt 960
Gly Ser Val Asn Phe Phe Thr Thr Trp Gln His Tyr Lys Ala Gly Phe
285 290 295 300

ggg cgg cca gac gga gaa tac tgg ctg ggc ctt gaa ccc gtg tat cag 1008
Gly Arg Pro Asp Gly Glu Tyr Trp Leu Gly Leu Glu Pro Val Tyr Gln
305 310 315

ctg acc agc cgt ggg gac cat gag ctg ctg gtt ctc ctg gag gac tgg 1056
Leu Thr Ser Arg Gly Asp His Glu Leu Leu Val Leu Leu Glu Asp Trp
320 325 330

ggg ggc cgt gga gca cgt gcc cac tat gat ggc ttc tcc ctg gaa ccc 1104
Gly Gly Arg Gly Ala Arg Ala His Tyr Asp Gly Phe Ser Leu Glu Pro
335 340 345

gag agc gac cac tac cgc ctg cgg ctt ggc cag tac cat ggt gat gct 1152
Glu Ser Asp His Tyr Arg Leu Arg Leu Gly Gln Tyr His Gly Asp Ala
350 355 360

gga gac tct ctt tcc tgg cac aat gac aag ccc ttc agc acc gtg gat 1200
Gly Asp Ser Leu Ser Trp His Asn Asp Lys Pro Phe Ser Thr Val Asp
365 370 375 380

agg gac cga gac tcc tat tct ggt aac tgt gcc ctg tac cag cgg gga 1248
Arg Asp Arg Asp Ser Tyr Ser Gly Asn Cys Ala Leu Tyr Gln Arg Gly
385 390 395

ggc tgg tgg tac cat gcc tgt gcc cac tcc aac ctc aac ggt gtg tgg 1296
Gly Trp Trp Tyr His Ala Cys Ala His Ser Asn Leu Asn Gly Val Trp
400 405 410

cac cac ggc ggc cac tac cga agc cgc tac cag gat ggt gtc tac tgg 1344
His His Gly Gly His Tyr Arg Ser Arg Tyr Gln Asp Gly Val Tyr Trp
415 420 425

gct gag ttt cgt ggt ggg gca tat tct ctc agg aag gcc gcc atg ctc 1392
Ala Glu Phe Arg Gly Gly Ala Tyr Ser Leu Arg Lys Ala Ala Met Leu
430 435 440

att cgg ccc ctg aag ctg tga 1413
Ile Arg Pro Leu Lys Leu
445 450

<210> 2

<211> 470

<212> PRT

<213> Homo sapiens

<400> 2

Met Gly Lys Pro Trp Leu Arg Ala Leu Gln Leu Leu Leu Leu Leu Gly
-20 -15 -10 -5

Ala Ser Trp Ala Arg Ala Gly Ala Pro Arg Cys Thr Tyr Thr Phe Val
-1 1 5 10

Leu Pro Pro Gln Lys Phe Thr Gly Ala Val Cys Trp Ser Gly Pro Ala
15 20 25

Ser Thr Arg Ala Thr Pro Glu Ala Ala Asn Ala Ser Glu Leu Ala Ala
30 35 40

Leu Arg Met Arg Val Gly Arg His Glu Glu Leu Leu Arg Glu Leu Gln
45 50 55 60

Arg Leu Ala Ala Ala Asp Gly Ala Val Ala Gly Glu Val Arg Ala Leu
65 70 75

Arg Lys Glu Ser Arg Gly Leu Ser Ala Arg Leu Gly Gln Leu Arg Ala
80 85 90

Gln Leu Gln His Glu Ala Gly Pro Gly Ala Gly Pro Gly Ala Asp Leu
95 100 105

Gly Ala Glu Pro Ala Ala Ala Leu Ala Leu Leu Gly Glu Arg Val Leu
110 115 120

Asn Ala Ser Ala Glu Ala Gln Arg Ala Ala Ala Arg Phe His Gln Leu
125 130 135 140

Asp Val Lys Phe Arg Glu Leu Ala Gln Leu Val Thr Gln Gln Ser Ser
145 150 155

Leu Ile Ala Arg Leu Glu Arg Leu Cys Pro Gly Gly Ala Gly Gly Gln
160 165 170

Gln Gln Val Leu Pro Pro Pro Pro Leu Val Pro Val Val Pro Val Arg
175 180 185

Leu Val Gly Ser Thr Ser Asp Thr Ser Arg Met Leu Asp Pro Ala Pro
190 195 200

Glu Pro Gln Arg Asp Gln Thr Gln Arg Gln Gln Glu Pro Met Ala Ser
205 210 215 220

Pro Met Pro Ala Gly His Pro Ala Val Pro Thr Lys Pro Val Gly Pro

225 230 235

Trp Gln Asp Cys Ala Glu Ala Arg Gln Ala Gly His Glu Gln Ser Gly

240 245 250

Val Tyr Glu Leu Arg Val Gly Arg His Val Val Ser Val Trp Cys Glu

255 260 265

Gln Gln Leu Glu Gly Gly Gly Trp Thr Val Ile Gln Arg Arg Gln Asp

270 275 280

Gly Ser Val Asn Phe Phe Thr Thr Trp Gln His Tyr Lys Ala Gly Phe

285 290 295 300

Gly Arg Pro Asp Gly Glu Tyr Trp Leu Gly Leu Glu Pro Val Tyr Gln

305 310 315

Leu Thr Ser Arg Gly Asp His Glu Leu Leu Val Leu Leu Glu Asp Trp

320 325 330

Gly Gly Arg Gly Ala Arg Ala His Tyr Asp Gly Phe Ser Leu Glu Pro

335 340 345

Glu Ser Asp His Tyr Arg Leu Arg Leu Gly Gln Tyr His Gly Asp Ala

350 355 360

Gly Asp Ser Leu Ser Trp His Asn Asp Lys Pro Phe Ser Thr Val Asp
365                 370                 375                 380

Arg Asp Arg Asp Ser Tyr Ser Gly Asn Cys Ala Leu Tyr Gln Arg Gly
                385                 390                 395

Gly Trp Trp Tyr His Ala Cys Ala His Ser Asn Leu Asn Gly Val Trp
            400                 405                 410

His His Gly Gly His Tyr Arg Ser Arg Tyr Gln Asp Gly Val Tyr Trp
        415                 420                 425

Ala Glu Phe Arg Gly Gly Ala Tyr Ser Leu Arg Lys Ala Ala Met Leu
    430                 435                 440

Ile Arg Pro Leu Lys Leu
445                 450

<210> 3
<211> 1412
<212> DNA
<213> Mus musculus

<220>
<221> CDS

<222> (1)..(1371)

<220>

<221> mat_peptide

<222> (73)..(1371)

<400> 3

atg ggg acc gcc agg cta cgc aag ctg caa ctg ctg ctt ctg ctg ggc     48

Met Gly Thr Ala Arg Leu Arg Lys Leu Gln Leu Leu Leu Leu Leu Gly

                -20                 -15                 -10

gct tgg agg gcg ctc gga ggt gcc gcg cgt tgc cgc gtc acc cta gtt     96

Ala Trp Arg Ala Leu Gly Gly Ala Ala Arg Cys Arg Val Thr Leu Val

            -5                  -1  1               5

ttg tcc ccg cag aag gca act agc gcc gtc tgc agg agc tca gag gcc     144

Leu Ser Pro Gln Lys Ala Thr Ser Ala Val Cys Arg Ser Ser Glu Ala

    10                  15                  20

acc caa gac agc gaa ctg gcc acg ctg cgc atg cgc ctg ggt cgc cac     192

Thr Gln Asp Ser Glu Leu Ala Thr Leu Arg Met Arg Leu Gly Arg His

25                  30                  35                  40

gag gag ctg ctg cgc gcg ctg caa agg cgt gcg gcg gag ggt ggt gcg     240

Glu Glu Leu Leu Arg Ala Leu Gln Arg Arg Ala Ala Glu Gly Gly Ala

                45                  50                  55

ctc gcg gac gag gtg cgc gca ctg cgc gag cac agt ctc acc ctg aac 288
Leu Ala Asp Glu Val Arg Ala Leu Arg Glu His Ser Leu Thr Leu Asn
60 65 70

acg cgc ctg ggc cag ctg cgc gcg caa ttg cag cag gag gcg agg gcg 336
Thr Arg Leu Gly Gln Leu Arg Ala Gln Leu Gln Gln Glu Ala Arg Ala
75 80 85

gag cct gac ctg ggg gcg gag cct gct gct gca ctt ggt ttg cta gcc 384
Glu Pro Asp Leu Gly Ala Glu Pro Ala Ala Ala Leu Gly Leu Leu Ala
90 95 100

gag cgc gcg ctg gac gct gag gcc gaa gcg cgc cgg acg acg gca cgc 432
Glu Arg Ala Leu Asp Ala Glu Ala Glu Ala Arg Arg Thr Thr Ala Arg
105 110 115 120

ctg cag cag ctg gac gca cag ctc cgt gag cat gcg cag ctc atg agc 480
Leu Gln Gln Leu Asp Ala Gln Leu Arg Glu His Ala Gln Leu Met Ser
125 130 135

cag cat agc agc ctc ctc ggc cgc ctg caa cgc gcg tgc gcg ggc ccg 528
Gln His Ser Ser Leu Leu Gly Arg Leu Gln Arg Ala Cys Ala Gly Pro
140 145 150

gaa cgg gga cag cag cag gtc ctg cca ctg ccc ctg gcg cct ctg gtg 576

Glu Arg Gly Gln Gln Gln Val Leu Pro Leu Pro Leu Ala Pro Leu Val
155 160 165

cct ctg agc ctc gtg ggc agt gcc agc aac acc agc agg agg ctg gac 624
Pro Leu Ser Leu Val Gly Ser Ala Ser Asn Thr Ser Arg Arg Leu Asp
170 175 180

caa act cca gag cac cag aga gag cag agc ttg aga cag cag ggg cct 672
Gln Thr Pro Glu His Gln Arg Glu Gln Ser Leu Arg Gln Gln Gly Pro
185 190 195 200

cca tct tct ctg ctg ccc aca ggg cac ctt gct gtc ccc aca agg cca 720
Pro Ser Ser Leu Leu Pro Thr Gly His Leu Ala Val Pro Thr Arg Pro
205 210 215

gtg ggc cca tgg agg gat tgt gca gag gct cac ggg gca ggt cac tgg 768
Val Gly Pro Trp Arg Asp Cys Ala Glu Ala His Gly Ala Gly His Trp
220 225 230

cag agt gga gtg tat gac ctg cgg ctg ggc cgt cgt gta gta gcc gtg 816
Gln Ser Gly Val Tyr Asp Leu Arg Leu Gly Arg Arg Val Val Ala Val
235 240 245

tgg tgt gaa cag cag cag gaa ggt gga ggc tgg act gtc atc cag aga 864
Trp Cys Glu Gln Gln Gln Glu Gly Gly Gly Trp Thr Val Ile Gln Arg
250 255 260

cgg cag gac ggc tct gtc aac ttc ttc acc aac tgg cag cac tac aag 912
Arg Gln Asp Gly Ser Val Asn Phe Phe Thr Asn Trp Gln His Tyr Lys
265 270 275 280

gcg ggc ttt ggg cgt cca gaa gga gaa tac tgg ctg ggc ctg gaa cct 960
Ala Gly Phe Gly Arg Pro Glu Gly Glu Tyr Trp Leu Gly Leu Glu Pro
285 290 295

gtg cat cag gtg aca agc cgt ggg gac cac gag ctg ctg ata ctc cta 1008
Val His Gln Val Thr Ser Arg Gly Asp His Glu Leu Leu Ile Leu Leu
300 305 310

gag gac tgg ggg ggc cgt gca gca cgc gcc cac tac gac agc ttc tcc 1056
Glu Asp Trp Gly Gly Arg Ala Ala Arg Ala His Tyr Asp Ser Phe Ser
315 320 325

ttg gag cct gag agt gac cac tac cgt ctg cgg ctt ggc cag tac cac 1104
Leu Glu Pro Glu Ser Asp His Tyr Arg Leu Arg Leu Gly Gln Tyr His
330 335 340

ggc gat gcc gga gac tcc ctc tct tgg cac aat gac aaa cct ttc agc 1152
Gly Asp Ala Gly Asp Ser Leu Ser Trp His Asn Asp Lys Pro Phe Ser
345 350 355 360

act gtg gat agg gac aga gac tca tat tct ggt aac tgt gcc ctg tac 1200

Thr Val Asp Arg Asp Arg Asp Ser Tyr Ser Gly Asn Cys Ala Leu Tyr
365 370 375

cat cgt ggg ggc tgg tgg tac cat gcc tgt gcc cac tct aac ctc aat 1248
His Arg Gly Gly Trp Trp Tyr His Ala Cys Ala His Ser Asn Leu Asn
380 385 390

gga gta tgg tat cat gga ggt cat tac cgg agc cga tac cag gac ggg 1296
Gly Val Trp Tyr His Gly Gly His Tyr Arg Ser Arg Tyr Gln Asp Gly
395 400 405

gtc tac tgg gcc gag ttc cgt ggt ggg gcg tac tct ctg aag aaa gct 1344
Val Tyr Trp Ala Glu Phe Arg Gly Gly Ala Tyr Ser Leu Lys Lys Ala
410 415 420

gtt atg ttg acc cgg ctt gtg cgc ttg tgactgtccc atcagtaccc 1391
Val Met Leu Thr Arg Leu Val Arg Leu
425 430

ccaagggtgt tcctatctcc t 1412

<210> 4
<211> 457
<212> PRT
<213> Mus musculus

<400> 4

Met Gly Thr Ala Arg Leu Arg Lys Leu Gln Leu Leu Leu Leu Leu Gly

                -20                 -15                 -10

Ala Trp Arg Ala Leu Gly Gly Ala Ala Arg Cys Arg Val Thr Leu Val

            -5                  -1  1               5

Leu Ser Pro Gln Lys Ala Thr Ser Ala Val Cys Arg Ser Ser Glu Ala

    10                  15                  20

Thr Gln Asp Ser Glu Leu Ala Thr Leu Arg Met Arg Leu Gly Arg His

25                  30                  35                  40

Glu Glu Leu Leu Arg Ala Leu Gln Arg Arg Ala Ala Glu Gly Gly Ala

                45                  50                  55

Leu Ala Asp Glu Val Arg Ala Leu Arg Glu His Ser Leu Thr Leu Asn

            60                  65                  70

Thr Arg Leu Gly Gln Leu Arg Ala Gln Leu Gln Gln Glu Ala Arg Ala

        75                  80                  85

Glu Pro Asp Leu Gly Ala Glu Pro Ala Ala Ala Leu Gly Leu Leu Ala

    90                  95                  100

Glu Arg Ala Leu Asp Ala Glu Ala Glu Ala Arg Arg Thr Thr Ala Arg
105                 110                 115                 120

Leu Gln Gln Leu Asp Ala Gln Leu Arg Glu His Ala Gln Leu Met Ser
                125                 130                 135

Gln His Ser Ser Leu Leu Gly Arg Leu Gln Arg Ala Cys Ala Gly Pro
            140                 145                 150

Glu Arg Gly Gln Gln Gln Val Leu Pro Leu Pro Leu Ala Pro Leu Val
        155                 160                 165

Pro Leu Ser Leu Val Gly Ser Ala Ser Asn Thr Ser Arg Arg Leu Asp
    170                 175                 180

Gln Thr Pro Glu His Gln Arg Glu Gln Ser Leu Arg Gln Gln Gly Pro
185                 190                 195                 200

Pro Ser Ser Leu Leu Pro Thr Gly His Leu Ala Val Pro Thr Arg Pro
                205                 210                 215

Val Gly Pro Trp Arg Asp Cys Ala Glu Ala His Gly Ala Gly His Trp
            220                 225                 230

Gln Ser Gly Val Tyr Asp Leu Arg Leu Gly Arg Arg Val Val Ala Val
        235                 240                 245

Trp Cys Glu Gln Gln Gln Glu Gly Gly Gly Trp Thr Val Ile Gln Arg
250 255 260
Arg Gln Asp Gly Ser Val Asn Phe Phe Thr Asn Trp Gln His Tyr Lys
265 270 275 280
Ala Gly Phe Gly Arg Pro Glu Gly Glu Tyr Trp Leu Gly Leu Glu Pro
285 290 295
Val His Gln Val Thr Ser Arg Gly Asp His Glu Leu Leu Ile Leu Leu
300 305 310
Glu Asp Trp Gly Gly Arg Ala Ala Arg Ala His Tyr Asp Ser Phe Ser
315 320 325
Leu Glu Pro Glu Ser Asp His Tyr Arg Leu Arg Leu Gly Gln Tyr His
330 335 340
Gly Asp Ala Gly Asp Ser Leu Ser Trp His Asn Asp Lys Pro Phe Ser
345 350 355 360
Thr Val Asp Arg Asp Arg Asp Ser Tyr Ser Gly Asn Cys Ala Leu Tyr
365 370 375
His Arg Gly Gly Trp Trp Tyr His Ala Cys Ala His Ser Asn Leu Asn

380 385 390

Gly Val Trp Tyr His Gly Gly His Tyr Arg Ser Arg Tyr Gln Asp Gly

395 400 405

Val Tyr Trp Ala Glu Phe Arg Gly Gly Ala Tyr Ser Leu Lys Lys Ala

410 415 420

Val Met Leu Thr Arg Leu Val Arg Leu

425 430

<210> 5

<211> 36

<212> DNA

<213> Artificial Sequence

<220>

<223> Description of Artificial Sequence:an artificially

synthesized primer sequence

<400> 5

agtctagaat ggggaccgcc aggctacgca agctgc 36

<210> 6

<211> 28

<212> DNA

<213> Mus musculus

<400> 6

aggagatagg aacacccttg ggggtact 28

<210> 7

<211> 17

<212> PRT

<213> Artificial Sequence

<220>

<223> Description of Artificial Sequence:mouse AGF

antigen

<400> 7

Asn Thr Ser Arg Arg Leu Asp Gln Thr Pro Glu His Gln Arg Glu Gln

1               5                   10                  15

Cys

<210> 8

<211> 32

<212> DNA
<213> Artificial Sequence

<220>
<223> Description of Artificial Sequence:an artificially
      synthesized linker sequence

<400> 8
caagcttatc gatgtcgacg cggccgcggt ac                                32

<210> 9
<211> 32
<212> DNA
<213> Artificial Sequence

<220>
<223> Description of Artificial Sequence:an artificially
      synthesized linker sequence

<400> 9
cgcggccgcg tcgacatcga taagcttggt ac                                32

<210> 10
<211> 20

<212> DNA
<213> Artificial

<220>
<223> an artificially synthesized primer sequence

<400> 10
gctcctgggc aacgtgctgg                                                    20

<210> 11
<211> 19
<212> DNA
<213> Artificial

<220>
<223> an artificially synthesized primer sequence

<400> 11
ctgctgtctc aagctctgc                                                     19

<210> 12
<211> 30
<212> DNA
<213> Artificial

<220>

<223> an artificially synthesized primer sequence

<400> 12

atggggaagc cctggctgcg tgcgctacag                                        30

<210> 13

<211> 30

<212> DNA

<213> Artificial

<220>

<223> an artificially synthesized primer sequence

<400> 13

tcacagcttc aggggccgaa tgagcatggc                                        30

<210> 14

<211> 31

<212> DNA

<213> Artificial

<220>

<223> an artificially synthesized primer sequence

<400> 14
cgggatccac cgggcgggcg ccccgcgctg c                                31

<210> 15
<211> 32
<212> DNA
<213> Artificial

<220>
<223> an artificially synthesized primer sequence

<400> 15
cgctcgagtc acagcttcag gggccgaatg ag                               32

<210> 16
<211> 31
<212> DNA
<213> Artificial

<220>
<223> an artificially synthesized primer sequence

<400> 16

cgggatccac agggcgctcg gaggtgccgc g                                31

<210> 17

<211> 32

<212> DNA

<213> Artificial

<220>

<223> an artificially synthesized primer sequence

<400> 17

cgctcgagtc acaagcgcac aagccgggtc aa                               32

<210> 18

<211> 97

<212> DNA

<213> Artificial Sequence

<220>

<223> double strand oligodeoxynucleotide

<400> 18

agcttgccac catgaagacg atcatcgccc tgagctacat cttctgcctg gtattcgccg 60

actacaagga ggatgatgac aaggggatcc actagtc 97

<210> 19

<211> 97

<212> DNA

<213> Artificial Sequence

<220>

<223> double strand oligodeoxynucleotide

<400> 19

tcgagactag tggatcccct tgtcatcatc ctccttgtag tcggcgaata ccaggcagaa 60

gatgtagctc agggcgatga tcgtcttcat ggtggca 97

## Description

### Technical Field

This invention relates to angiopoietin-related growth factors (hereinafter abbreviated as AGFs), genes encoding the AGFs, vectors comprising the genes, and host cells comprising the vectors. This invention also relates to transgenic animals manipulated to express the AGFs in epidermis, and applications of the AGFs or the polynucleotides encoding the AGFs.

### Background Art

To date, approximately ten members of angiopoietin family have been known identified, based on their structural similarity. Angiopoietin-1 and angiopoietin-2, which are ligands for tyrosine kinase receptor Tie2 that is specifically expressed in vascular endothelial cells and in some blood cells, have been the most extensively studied. Angiopoietin-1 and angiopoietin-2 are known to be involved in angiogenesis through activities such as promotion of cell adhesion, cell chemotaxis, and cell survival (Gale N.W. and Yancopoulos G.D., Genes&Dev. 13 (9) : 1055-1066 (1999)).

For other members, *i*.*e*., Angiopoietin Related Protein-1 (ARP-1), Angiopoietin Related Protein-2 (ARP-2), NL8/NEW, fasting-induced adipose factor / PPAR γ angiopoietin factor (fasting-induced adipose factor / peroxisome proliferator activated receptor γ angiopoietin-related (FIAR/PGAR)), and AngPTL3, receptors have not been identified yet, and little known about their physiological functions.

NL8/NEW is known to confer tumorigenicity to CHO cells when CHO cells stably expressing the NL8/NEW are grafted under the skin of nude mice, and also known to be amplified in various cancers (W099/15653). It has been proposed that NL8/NEW is likely to be a member of the angiopoietin family involved in angiogenesis based on the fact that it has 28.8 and 27.2% amino acid sequence homology to Ang-1 and Ang-2, respectively, and that the sequence is conserved at least 40% in the fibrinogen domain at the C terminal (Unexamined Published Japanese Patent Application No. (JP-A) 2000-300263). However, to date, no members of the angiopoietin family have been shown to be able to proliferate the epidermal cells and regenerate tissues.

Wounds can generally heal without any therapeutic intervention. However, many diseases, such as diabetes, artery obstructive diseases, psoriasis, atopic dermatitis, contact dermatitis, Crohn disease, epidermolysis bullosa, skin aging, and innervation diseases, can cause delayed wound healing, which can result in chronic wounds (Cruse P.J. and Foord R., Arch. Surg. 107(2): 206-210 (1973); Schrock T.R. *et al*., Ann. Surg. 177(5): 513-518 (1973) ; Poole G.U.Jr., Surgery 97 (6) : 631-640 (1985); Irvin G.L. *et al*., Am. Surg. 51(7): 418-422 (1985)). Accordingly, promotion of the natural wound healing process is desired in many cases in order to reduce the risk of bacterial infection and the rest period of the patient.

In the repair process of skin loss, such as wound healing, acute inflammation associated with angiogenesis occurs in the early stages. Cell proliferation and differentiation, as well as formation of extracellular matrix, occur in the middle stages. Finally, skin composition is reconstructed in the late stages (Peacock E.E.Jr., Wound Repair, 2nd edition, WB SAunders, Philadelphia (1984)). At the wound site, epidermal cells, fibroblasts, and inflammatory cells are interrelated with each other. Although angiogenesis factors, cell chemotactic factors, cell growth factors, cell differentiation factors, and the like have been implicated in the wound healing process, the details remain unknown. Therefore, few treatments have been developed for inhibiting wound healing disorders.

Current wound treatments are limited to the use of external medicines, such as solutions and ointments, or bandages and covering materials, such as sticking plasters for sterilization or cleaning of the lesion, and transplants of skin tissues, cultured skin cells, and/or matrix proteins. To date, protein factors, such as keratinocyte growth factor-1 (KGF-1), KGF-2, platelet-derived growth factor (PDGF), basic FGF, acidic FGF, EGF, TGF-α, TGF-β, NDF, and IGF-1, have been identified as involved in the wound healing, and some of these factors have been recently put in clinical use for improving wound healing. However, despite such attempts, use of such factors has failed to decisively improve upon conventional treatments.

In mammals, including humans, regeneration of highly differentiated tissues with complicated structure as found in some amphibians is difficult (Brockes J.P. *et al*., Nat Rev Mol Cell Biol. 2002, 3(8): 566-574). However, attempts have been made in regenerative medicine not only to regenerate organs but also to regenerate tissues and recover the function of the parts lost from the body by exterior injury, inflammation, tumor, extraction, or the like, for both hard tissues, such as bone tissue and cartilage tissue, and soft tissues, such as epithelial tissue, connective tissue, and nerve tissue.

Regeneration of tissues involves cell proliferation, and use of protein factors having cell proliferative activity as tissue regenerative agents has been investigated. For example, basic FGF has proliferative activity for fibroblasts, and hence granulation activity in the course of wound healing; accordingly, it is used as a wound healing promoting agent. However, its activity is limited, and it is incapable of repairing, for example, the ear punch hole of a mouse where the tissue has been completely lost.

Development of a therapeutic agent having regenerative activity for cartilage tissue has been highly awaited for use in treating osteoarthritis and other diseases of joints caused by aging or sport injuries. Since osteoarthritis is associated with the loss of cartilage tissue (Onstott A.T. *et al*., AORN J., 71(4), 843-845, 848-851, 2000), a factor having a proliferative activity for chondrocytes or regenerative activity for cartilage tissue would be a candidate agent for treating osteoarthritis (Buckwalter J.A. *et al*., Instructional Course Lectures, 47, 487-504, 1998). Cartilage is an organ known to be extremely hard to regenerate. Despite a number of different approaches that have been taken for the regeneration of the cartilage, so far, there are no reports of successful regeneration of cartilage in a simple way.

Mast cells are cells found in tissues and feature granules present in the cytoplasm. When mast cells are activated by allergic reaction or other stimulations, they release various mediators involved in the biological defense mechanism and symptoms of various diseases are thereby induced. Mast cells are involved in many diseases including acute allergic diseases, chronic allergic diseases, and chronic inflammatory diseases. For such diseases, the presence in the mast cell granules of histamine, proteases, proteoglycan, cytokines, chemokines, and growth factors, and association of these mediators in the onset of various diseases and progress of symptoms have been indicated (Dean D. Metcalfe *et al*., Physiol. Reviews 77(4): 1033-1079 (1997)). For example, in psoriasis vulgaris, significant thickening of epidermal cell as well as proliferation and activation of mast cells have been observed (Petersen L.J. *et al*., Acta Dermato-Venerologica 78 (3) : 190-193 (1998)). Mast cells are also known to play an important role in the tissue repair. Also, degranulation of the mast cells at wound sites has been reported, which indicates the association of the components in the granule with the promotion of wound healing (Experomental Dermatology, 8(1): 1-16, 1999).

A disease model animal can be used not only for elucidating the pathological cause(s) of the disease but also in the screening of a therapeutic agent for the disease. In particular, various transgenic mice have been developed for use as skin disease models. In a transgenic mouse utilizing the K14 promoter, an exogenous gene can be expressed in the epidermal cells of the basal layer. For example, a psoriasis-like transgenic mouse having thickened epidermal cells has been produced by expressing TGF-α using the K14 promoter (Genes Dev. 5: 714-727 (1991)), and an atopic dermatitis-like transgenic mouse has been produced by expressing interleukin-4 using the K14 promoter (J. Invest. Dermatol. 117(4): 977-983 (2001)).
Reference 1: Gale N.W. and Yancopoulos G.D., Genes&Dev. 13(9): 1055-1066 (1999)
Reference 2: Cruse P.J. and Foord R., Arch. Surg. 107(2): 206-210 (1973)
Reference 3: Schrock T.R. *et al*., Ann. Surg. 177(5): 513-518 (1973)
Reference 4: Poole G.U.Jr., Surgery 97(6): 631-640 (1985)
Reference 5: Irvin G.L. *et al*., Am. Surg. 51(7): 418-422 (1985)
Reference 6: Peacock E.E.Jr., Wound Repair, 2nd edition, WB SAunders, Philadelphia (1984)
Reference 7: Brockes J.P. *et al*., Nat Rev Mol Cell Biol., 3(8):566-574 (2002)
Reference 8: Onstott A.T. *et al*., AORN J., 71(4), 843-845, 848-851 (2000)
Reference 9: Buckwalter J.A. *et al*., Instructional Course Lectures, 47, 487-504 (1998)
Reference 10: Dean D. Metcalfe *et al*., Physiol. Reviews 77(4): 1033-1079 (1997)
Reference 11: Petersen L.J. *et al*., Acta Dermato-Venerologica 78(3): 190-193 (1998)
Reference 12: Experomental Dermatology, 8(1): 1-16 (1999)
Reference 13: Genes Dev. 5(5): 714-727 (1991)
Reference 14: J. Invest. Dermatol. 117(4): 977-983 (2001) Patent reference 1: WO99/15653
Patent reference 2: Unexamined Published Japanese Patent Application No. 2000-300263

### Disclosure of the Invention

An objective of the present invention is to provide polypeptides having epidermal cell proliferating activity. Another objective of the present invention is to provide polynucleotides encoding the polypeptides, and methods for producing them. A further objective of the present invention is to provide transgenic animals wherein expression of the polypeptides is regulated, and applications thereof. A still further objective of the present invention is to provide wound healing agents or tissue regenerative agents utilizing the polypeptides.

The present inventors obtained a mouse ortholog gene of human NL8/NEW. When the distribution of expression was analyzed in mice and humans, it was confirmed that these genes are specifically expressed in livers. Next, the present inventors produced transgenic (Tg) mice expressing the mouse NL8/NEW gene in the epidermal cells. These Tg mice exhibited marked thickening of the epidermal cell layer and an increased proliferative activity in the cells of the thickened epidermis. The present inventors noticed that the characteristics observed in these Tg mice are quite similar to symptoms of patients suffering from skin psoriasis. Unexpectedly, the Tg mice showed an enhanced vascular permeability in addition to the enhanced angiogenesis. By reacting the recombinant mouse NL8/NEW with the isolated mouse epidermal cells, it was confirmed that the epidermal cells were induced to proliferate by the recombinant mouse NL8/NEW, and, using a wound healing model, it was confirmed that the Tg animals promoted the wound healing. Based on these findings, the mouse ortholog of the human NL8/NEW obtained was designated "mouse angiopoietin-related growth factor (AGF)", and the human NL8/NEW was designated human AGF.

In addition, the present inventors have also found that the AGF proteins have a strong tissue regenerative activity. The tissue regenerative activity of the AGF proteins was so strong that not only the epidermal tissue but also the dermal tissue, subcutaneous tissue, and cartilage tissue were regenerated.

Accordingly, the present invention relates to the following polypeptides, the polynucleotides encoding the polypeptides, the non-human vertebrates comprising regulated expression of the polypeptides, and the use thereof.
[1] A polypeptide of any one of the following (1) to (4):
   (1) a polypeptide comprising the amino acid sequence of 1 to 433 of SEQ ID NO: 4, wherein said polypeptide has epidermal cell proliferating activity;
   (2) a polypeptide consisting of the amino acid sequence of 1 to 433 of SEQ ID NO: 4, wherein substitution, deletion, and/or insertion of one to several amino acid residues has occurred at one to several sites of the amino acid sequence, wherein said polypeptide has epidermal cell proliferating activity;
   (3) a polypeptide encoded by a DNA which hybridizes with the polynucleotide shown in the nucleotide sequence of 73 to 1371 of SEQ ID NO: 3 under stringent conditions, wherein said polypeptide has epidermal cell proliferating activity; and
   (4) a polypeptide consisting of an amino acid sequence having a homology of at least 95% to the amino acid sequence of 1 to 433 of SEQ ID NO: 4, wherein said polypeptide has epidermal cell proliferating activity.
[2] A polypeptide of one of the following (1) or (2):
   (1) a polypeptide consisting of the amino acid sequence of 1 to 433 of SEQ ID NO: 4; and
   (2) a polypeptide consisting of the amino acid sequence of SEQ ID NO: 4.
[3] A polynucleotide encoding the polypeptide of [1] or [2].
[4] A vector comprising the polynucleotide of [3] in an expressible form.
[5] A host cell comprising the vector of [4].
[6] A non-human transgenic animal manipulated to express the polypeptide of [1] or [2] in its epidermis.
[7] An animal model for psoriasis comprising a non-human transgenic animal manipulated to express in its epidermis a polypeptide of any one of the following (a) to (d) :
   (a) a polypeptide comprising the amino acid sequence of 1 to 450 of SEQ ID NO: 2 or the amino acid sequence of 1 to 433 of SEQ ID NO: 4, wherein said polypeptide has epidermal cell proliferating activity;
   (b) a polypeptide comprising the amino acid sequence of 1 to 450 of SEQ ID NO: 2 or the amino acid sequence of 1 to 433 of SEQ ID NO: 4, wherein substitution, deletion, and/or insertion of one to several amino acid residues has occurred at one to several sites of said amino acid sequence, wherein said polypeptide has epidermal cell proliferating activity;
   (c) a polypeptide encoded by a DNA which hybridizes with the DNA encoding the amino acid sequence of 1 to 450 of SEQ ID NO: 2 or the amino acid sequence of 1 to 433 of SEQ ID NO: 4 under stringent conditions, wherein said polypeptide has epidermal cell proliferating activity; and
   (d) a polypeptide consisting of an amino acid sequence having a homology of at least 95% to the amino acid sequence of 1 to 450 of SEQ ID NO: 2 or to the amino acid sequence of 1 to 433 of SEQ ID NO: 4, wherein said polypeptide has epidermal cell proliferating activity.
[8] A tissue regenerative agent comprising as its active ingredient a polypeptide of any one of the following (a) to (d) :
   (a) a polypeptide comprising the amino acid sequence of 1 to 450 of SEQ ID NO: 2 or the amino acid sequence of 1 to 433 of SEQ ID NO: 4, wherein said polypeptide has epidermal cell proliferating activity;
   (b) a polypeptide comprising the amino acid sequence of 1 to 450 of SEQ ID NO: 2 or the amino acid sequence of 1 to 433 of SEQ ID NO: 4, wherein substitution, deletion, and/or insertion of one to several amino acid residues has occurred at one to several sites of said amino acid sequence, wherein said polypeptide has epidermal cell proliferating activity;
   (c) a polypeptide encoded by a DNA which hybridizes with the DNA encoding the amino acid sequence of 1 to 450 of SEQ ID NO: 2 or the amino acid sequence of 1 to 433 of SEQ ID NO: 4 under stringent conditions, wherein said polypeptide has epidermal cell proliferating activity; and
   (d) a polypeptide consisting of an amino acid sequence having a homology of at least 95% to the amino acid sequence of 1 to 450 of SEQ ID NO: 2 or to the amino acid sequence of 1 to 433 of SEQ ID NO: 4, wherein said polypeptide has epidermal cell proliferating activity.
[9] The tissue regenerative agent according to [8], wherein the tissue is selected from the group consisting of skin tissue, epithelial tissue, cartilage tissue, and connective tissue.
[10] The tissue regenerative agent according to [8] or [9], wherein the tissue regeneration is wound healing.
[11] The tissue regenerative agent according to [8] or [9], wherein the tissue regenerative agent is a therapeutic agent for osteoarthritis.
[12] A tissue regenerative agent comprising as its active ingredient a polynucleotide of any one of the following (e) to (h) :
   (e) a polynucleotide comprising the nucleotide sequence of 61 to 1410 of SEQ ID NO: 1 or the nucleotide sequence of 73 to 1371 of SEQ ID NO: 3, wherein said polynucleotide encodes a polypeptide having epidermal cell proliferating activity;
   (f) a polynucleotide encoding a polypeptide comprising the amino acid sequence of 1 to 450 of SEQ ID NO: 2 or the amino acid sequence of 1 to 433 of SEQ ID NO: 4, wherein substitution, deletion, and/or insertion of one to several amino acid residues has occurred at one to several sites of said amino acid sequence, wherein said polypeptide has epidermal cell proliferating activity;
   (g) a polynucleotide which hybridizes with the DNA shown in the nucleotide sequence of 61 to 1410 of SEQ ID NO: 1 or the nucleotide sequence of 73 to 1371 of SEQ ID NO: 3 under stringent conditions, wherein said polynucleotide encodes a polypeptide having epidermal cell proliferating activity; and
   (h) a polynucleotide encoding a polypeptide consisting of an amino acid sequence having a homology of at least 95% to the amino acid sequence of 1 to 450 of SEQ ID NO: 2 or to the amino acid sequence of 1 to 433 of SEQ ID NO: 4, wherein said polypeptide has epidermal cell proliferating activity.
[13] The tissue regenerative agent according to [12], wherein the tissue is selected from the group consisting of skin tissue, epithelial tissue, cartilage tissue, and connective tissue.
[14] The tissue regenerative agent according to [12] or [13], wherein the tissue regeneration is wound healing.
[15] The tissue regenerative agent according to [12] or [13], wherein the tissue regenerative agent is a therapeutic agent for osteoarthritis.

The present invention relates to a polypeptide of any one of the following (1) to (4):
(1) a polypeptide comprising the amino acid sequence of 1 to 433 of SEQ ID NO: 4, wherein said polypeptide has epidermal cell proliferating activity;
(2) a polypeptide consisting of the amino acid sequence of 1 to 433 of SEQ ID NO: 4, wherein substitution, deletion, and/or insertion of 1 to 10 amino acid residues has occurred, wherein said polypeptide has epidermal cell proliferating activity (hereinafter referred to as an "equivalent" to a polypeptide consisting of the amino acid sequence from 1 to 433 of SEQ ID NO: 4);
(3) a polypeptide encoded by a DNA which hybridizes with the polynucleotide shown by the nucleotide sequence of 73 to 1371 of SEQ ID NO: 3 under "stringent conditions", wherein said polypeptide has epidermal cell proliferating activity (hereinafter referred to as a "hybridization equivalent" to a polypeptide consisting of the amino acid sequence from 1 to 433 of SEQ ID NO: 4); and
(4) a polypeptide consisting of an amino acid sequence having a homology of at least 95% to the amino acid sequence of 1 to 433 of SEQ ID NO: 4, wherein said polypeptide has epidermal cell proliferating activity (hereinafter referred to as a "homologous equivalent" to a polypeptide consisting of the amino acid sequence from 1 to 433 of SEQ ID NO: 4).

The amino acid sequence shown in SEQ ID NO: 4 is the amino acid sequence of the mouse AGF. The mouse AGF has a signal sequence (-20 to -1) on the N terminal that is cleaved when the mouse AFG is secreted to the exterior of the cells. Mature mouse AGF, produced as a result of this signal sequence cleavage and consisting of amino acids of 1 to 433 of SEQ ID NO: 4, has the relevant physiological activity. Therefore, the polypeptides of the present invention are polypeptides consisting of amino acids of 1 to 433 of SEQ ID NO: 4 or their "equivalents".

Preferred "equivalents" of the polypeptides consisting of the amino acid sequence of 1 to 433 of SEQ ID NO: 4 include polypeptides in which substitution, deletion, and/or insertion of preferably 1 to 10, more preferably 1 to 7, and even more preferably 1 to 5 amino acids has occurred in the amino acid sequence of 1 to 433 of SEQ ID NO: 4, so long as the resulting polypeptide retains an activity of proliferating epidermal cells, namely "AGF activity", identical to that of the polypeptide consisting of the amino acid sequence of 1 to 433 of SEQ ID NO: 4.

The amino acid to be substituted is preferably an amino acid having characteristics similar to that of the original amino acid sufficient to retain the function of the original polypeptide. For example, amino acids belonging to each of the following groups have characteristics similar to those of other members of the group. When these amino acids are substituted for other amino acids within the same group, the essential function of the protein is often retained. Such amino acid substitution is called conservative substitution, and it is known as a method for changing an amino acid sequence while retaining the function of the original polypeptide.
Nonpolar amino acids: Ala, Val, Leu, Ile, Pro, Met, Phe, and Trp;
Uncharged amino acids: Gly, Ser, Thr, Cys, Tyr, Asn, and Gln;
Acidic amino acids: Asp and Glu; and
Basic amino acids: Lys, Arg, and His.

The activity of a particular polypeptide for proliferating epidermal cells can be confirmed, for example, by the method described in Example 13, namely, by confirming whether the polypeptide of interest has the same activity as said polypeptide, using the proliferation of epidermal cells as an index.

In the present invention, "stringent conditions", such as those used to obtain an above hybridization equivalent, include hybridization conditions such as "5x SSPE, 5x Denhard's solution, 0.5% SDS, 40% formamide, and 200 µg/ml salmon sperm DNA, at 37°C overnight", and more stringent conditions such as "5x SSPE, 5x Denhard's solution, 0.5% SDS, 50% formamide, and 200 µg/ml salmon sperm DNA, at 42°C overnight". Washing conditions include mild conditions such as "5x SSC and 1% SDS at 42°C", usual conditions such as "0.5x SSC and 0.1% SDS at 42°C", and more stringent conditions such as "0.2x SSC and 0.1% SDS at 65°C".

The amino acid sequence of a "homologous equivalent" of the polypeptide consisting of the amino acid sequence of 1 to 433 of SEQ ID NO: 4 has a homology of at least 95%, and more preferably at least 97% to the amino acid sequence of 1 to 433 of SEQ ID NO: 4. The homology of amino acid sequences can be identified by using BLAST search under the conditions (parameters) described below.

In the present invention, the "equivalent" of the polypeptide consisting of the amino acid sequence of 1 to 433 of SEQ ID NO: 4, the "hybridization equivalent" of the polypeptide consisting of the amino acid sequence of 1 to 433 of SEQ ID NO: 4, and the "homologous equivalent" of the polypeptide consisting of the amino acid sequence of 1 to 433 of SEQ ID NO: 4 are together referred to as "equivalents". Preferred polypeptides of the present invention are polypeptides consisting of the amino acid sequence of 1 to 433 of SEQ ID NO: 4.

The source of the polypeptides of the present invention is not limited to mice. So long as the polypeptide is any one of the polypeptides of the above (1) to (4), for example, polypeptides from organisms other than mice, as well as artificial polypeptides genetically modified on the basis of the sequence of 1 to 433 of SEQ ID NO: 4, are also within the scope of polypeptides of the present invention.

The polypeptides of the present invention are preferably recombinant polypeptides.

The present invention includes any polynucleotide, so long as it comprises a nucleotide sequence encoding a polypeptide of the present invention, namely, is a polynucleotide comprising a nucleotide sequence encoding AGF defined by the amino acid sequence of 1 to 433 of SEQ ID NO: 4 or one of its "equivalents", and more specifically, comprises a nucleotide sequence encoding a polypeptide selected from the polypeptides of the present invention as described in the above (1) to (4). Preferred polynucleotides are polynucleotides consisting of the nucleotide sequence encoding the amino acid sequence of 1 to 433 of SEQ ID NO: 4, and more preferably, polynucleotides consisting of the nucleotide sequence of SEQ ID NO: 3, or nucleotides 73 to 1371 of SEQ ID NO: 3. In the context of the present invention, the term "polynucleotides" encompass both DNA and RNA molecules, preferably DNA.

The following 1) to 6) are provided to describe the polynucleotides of the present invention, the vectors of the present invention, the host cells of the present invention, the polypeptides of the present invention, the Tg animals according to the present invention, the animal models for psoriasis, the tissue regenerative agents, the wound healing agents, and the therapeutic agents for osteoarthritis of the present invention.

### 1) Methods for producing the polynucleotides of the present invention:

Methods for producing the polynucleotides of the present invention are not limited to any particular method, and exemplary methods include: methods utilizing PCR as described, for example, in WO02/052000; conventional genetic engineering methods (*i*.*e*., selecting transformants harboring desired cDNA from cells transformed with a cDNA library); and chemical synthesis methods. These methods will be described one by one.

### a) Production method 1

mRNA is extracted from cells or tissues capable of producing the polypeptides of the present invention. Next, two primers are designed to flank AGF mRNA or a part thereof for using the mRNA as the template. AGF cDNA or a part thereof can be obtained by reverse transcriptase-polymerase chain reaction (hereinafter referred to as RT-PCR). The polypeptides of the present invention can be produced by incorporating the AGF cDNA or part thereof thus obtained into an appropriate expression vector to express it in host cells.

For example, mRNA containing the mRNA encoding the polypeptides of the present invention is extracted by a known method from cells or tissues, such as mouse livers or embryos, which are capable of producing the protein. Exemplary extraction methods include the guanidine thiocyanate-hot phenol method and the guanidine thiocyanate-guanidine hydrochloride method, preferably the guanidine thiocyanate-cesium chloride method. The cells or tissues capable of producing the polypeptides can be identified by Northern blotting, using polynucleotides comprising nucleotide sequences encoding the polypeptides or a part thereof; or by Western blotting, using antibodies specific to the polypeptides, etc. The mRNA can be purified by a method commonly used in the art, for example, by adsorbing the mRNA on an oligo(dT) cellulose column and eluting the mRNA therefrom. The mRNA may also be further fractionated by sucrose density gradient centrifugation and such. Instead of extracting the mRNA, a commercially available pre-extracted mRNA may also be used.

Next, purified mRNA is subjected to the reverse transcriptase reaction in the presence of random primers or oligodT primers to synthesize first strand cDNA. This synthesis can be conducted by a method commonly used in the art. By using the first strand cDNA obtained and two primers flanking a part of the target gene, PCR is performed to amplify the target DNA of AGF. The resulting DNA is fractionated by agarose gel electrophoresis or the like. If desired, the DNA can be cleaved by restriction enzyme(s) or the like and then ligated to obtain the target DNA fragment.

### b) Production method 2

In addition to the method described above, polynucleotides of the present invention can be produced using genetic engineering methods commonly used in the art. First, single stranded cDNA is synthesized using a reverse transcriptase and the mRNA produced in the method described above as the template. Double stranded cDNA is then synthesized from this single stranded cDNA by a method such as the S1 nuclease method (Efstratiadis, A. *et al*., Cell 7: 279-288 (1976)), Land method (Land, H. *et al*., Nucleic Acids Res. 9: 2251-2266 (1981)), O. Joon Yoo method (Yoo, O.J. *et al*., Proc. Natl. Acad. Sci. USA 79: 1049-1053 (1983)), or Okayama-Berg method (Okayama, H. and Berg, P., Mol. Cell. Biol. 2: 161-170 (1982)).

Next, a recombinant plasmid produced by the method as described above is introduced into *E. coli*, such as the DH5α strain, and transformants are selected, using the resistance to tetracycline or ampicillin, for example, as an index. The transformation of host cells can be accomplished, for example, when the host cells are *E. coli*, by the method of Hanahan (Hanahan, D., J. Mol. Biol. 166: 557-580 (1983)), namely, by adding the recombinant DNA to competent cells prepared in the presence of CaCl₂, MgCl₂, or RbCl. In addition to plasmid vectors, phage vectors, such as lambda phage vectors, can also be used.

From the transformants thus obtained, strains carrying DNA encoding the target polypeptide(s) can be selected by methods known in the art, including transformant-screening methods using synthetic oligonucleotide probes, transformant-screening methods using probes produced by PCR, methods of screening for the polypeptides of the present invention produced in other animal cells, and selection methods using antibodies against the polypeptides of the present invention.

DNA encoding the polypeptides of the present invention can be collected from target transformants obtained according to a method known in the art (*e*.*g*., Maniatis, T. *et al*. (1982): "Molecular Cloning-A Laboratory Manual" Cold Spring Harbor Laboratory, NY). For example, the DNA may be collected by separating the fraction corresponding to the plasmid DNA from the cells, and cleaving the cDNA region from the plasmid DNA.

### c) Production method 3

The polynucleotides comprising a nucleotide sequence encoding an amino acid sequence represented by SEQ ID NO: 2 or SEQ ID NO: 4 can also be produced by ligating DNA fragments produced by chemical synthesis. Each DNA can be synthesized using a DNA synthesizer (for example, Oligo 1000M DNA Synthesizer (Beckman) or 394 DNA/RNA Synthesizer (Applied Biosystems)).

### d) Production method 4

Substances produced by genetic engineering means utilizing the polynucleotides of the present invention need not necessarily include all of the amino acid sequence of 1 to 433 of SEQ ID NO: 4 in order to exhibit the requisite function of the polypeptides of the present invention. For example, partial sequences of such polypeptides and polypeptides comprising additional amino acid sequences are also included in the polypeptides of the present invention as long as they show "AGF activity".

Furthermore, it is generally believed that eukaryotic genes exhibit polymorphisms, such as known for the interferon gene (see, *e*.*g*., Nishi, T. *et al*., J. Biochem. 97: 153-159 (1985)) and the like, and due to such polymorphisms, one or more amino acids may be substituted. Therefore, polypeptides comprising one to several amino acid substitutions, deletions, and/or insertions at one to several sites in the amino acid sequence of 1 to 433 of SEQ ID NO: 4 are likely to exhibit "AGF activity". As described above, these polypeptides are "equivalents" of the polypeptides consisting of the amino acid sequence of 1 to 433 of SEQ ID NO: 4, accordingly, they are within the scope of the present invention.

The present invention encompasses polynucleotides comprising the nucleotide sequences encoding such "equivalents". Such polynucleotides of the present invention may be produced by chemical synthesis of the nucleic acids by methods commonly used in the art, for example, by the phosphite triester method (Hunkapiller, M. *et al*., Nature 10: 105-111 (1984)) based on the information regarding the AGF of the present invention as described above. Codons for desired amino acids are known themselves, and any codon may be selected, for example, considering the codon frequency of the host utilized according to methods commonly used in the art (Crantham, R. *et al*., Nucleic Acids Res. 9: r43-r74 (1981)). In addition, nucleotide sequence codons can partially be changed according to methods commonly used in the art, for example, by site specific mutagenesis using primers comprising synthetic oligonucleotides encoding desired changes (Mark, D. F. *et al*., Proc. Natl. Acad. Sci. USA 81: 5662-5666 (1984)).

Other embodiments of the method for producing the "equivalents" of the present invention include those utilizing hybridization or gene amplification techniques. More specifically, those skilled in the art can routinely isolate DNA having a high homology to a DNA encoding a polypeptide of the present invention from DNA samples obtained from organisms of the same or different species, based on the nucleotide sequence (SEQ ID NO: 3) of the DNA encoding the polypeptide of the present invention or a part thereof, using hybridization technology (Current Protocols in Molecular Biology edit. Ausubel *et al*. (1987) Publish. John Wiley & Sons Section 6.3-6.4) to 'obtain proteins functionally equivalent to the polypeptide consisting of the amino acid sequence of 1 to 433 of SEQ ID NO: 4, namely proteins exhibiting the "AGF activity".

Thus, the present invention also encompasses polypeptides encoded by DNA which hybridizes with a DNA encoding a polypeptide consisting of the amino acid sequence of 1 to 433 of SEQ ID NO: 4, exhibiting "AGF activity", and a DNA encoding such polypeptides. Such DNA and polypeptides are preferably DNA which hybridizes under "stringent conditions" and polypeptide encoded by such DNA, respectively, and DNA which hybridizes with the DNA of the nucleotide sequence of 73 to 1371 of SEQ ID NO: 3 and the polypeptide encoded by such DNA.

Organisms used for isolating the DNA encoding such proteins include, but are not limited to, monkeys, pigs, bovines, and dogs, as well as mice.

Stringent hybridization conditions used for isolating the DNA encoding the "hybridization equivalents" of the polypeptide consisting of the amino acid sequence of 1 to 433 of SEQ ID NO: 4 include conditions such as "5x SSPE, 5x Denhard's solution, 0.5% SDS, 40% formamide, and 200 µg/ml salmon sperm DNA, at 37°C overnight", and more stringent conditions such as "5x SSPE, 5x Denhard's solution, 0.5% SDS, 50% formamide, and 200 µg/ml salmon sperm DNA, at 42°C overnight". Washing conditions include mild conditions such as "5x SSC and 1% SDS at 42°C", typical conditions such as "0.5x SSC and 0.1% SDS at 42°C", and more stringent conditions such as "0.2x SSC and 0.1% SDS at 65°C". When using higher stringency conditions as described above, isolation of DNA having a higher homology to the probe sequence can be expected. The above combinations of SSC, SDS, formamide, and temperature conditions, however, are examples, and those skilled in the art will realize that a stringency similar to that described above may be obtained by adequately combining the above factors or other factors (*e.g.*, probe concentration, probe length, and hybridization reaction time) which determine the hybridization stringency.

Polypeptides encoded by DNA isolated by such hybridization techniques generally have amino acid sequences that have a high homology to the amino acid sequence of 1 to' 433 of SEQ ID NO: 4. The term "high homology" indicates a sequence homology of at least 80%, preferably at least 90%, more preferably at least 95%, and even more preferably at least 97%. The homology of amino acid sequences may be determined by an algorithm of the BLAST search, and more specifically, can be calculated using the bl2seq program of the BLAST package (sgi 32 bit version, ver. 2.0.12, available from NCBI) (Tatiana A. Tatusova, Thomas L. Madden, FEMS Microbiol Lett. 174: 247-250 (1999)) and the default parameters. The parameters for pairwise alignment include the program name, blastp; Gap insertion cost value, 0; Gap elongation cost value, 0; Query sequence filter, SEG; and matrix, BLOSUM62.

Polypeptides functionally equivalent to the AGF polypeptide consisting of the amino acid sequence of 1 to 433 of SEQ ID NO: 4 may be obtained using gene amplification technology (PCR) (Current protocols in Molecular Biology edit. Ausubel *et al*. (1987) Publish. John Wiley & Sons Section 6.1-6.4) by designing primers based on parts of the nucleotide sequence (SEQ ID NO: 3) of the DNA encoding the AGF polypeptide of the present invention, and isolating DNA fragments comprising nucleotide sequences having a high homology to the nucleotide sequence of the DNA encoding the polypeptide consisting of the amino acid sequence of 1 to 433 of SEQ ID NO: 4. Preferred primers include, for example, oligonucleotides consisting of the nucleotide sequences of SEQ ID NO: 5 and SEQ ID NO: 6. An exemplary method using these primers is described in Example 1.

Sequencing of DNA produced by the above a) to d) can be accomplished, for example, by the chemical modification method of Maxam and Gilbert (Maxam, A.M. and Gilbert, W. (1980): "Methods in Enzymology" 65, 499-559) and dideoxynucleotide chain termination method using M13 (Messing J. and Vieira J., Gene 19: 269-276 (1982)).

The vectors, host cells, and AGF proteins of the present invention can be produced by the methods described below.

### 2) Production methods of vectors, host cells, and proteins of the present invention:

Isolated fragments comprising the genes encoding the polypeptides of the present invention can be incorporated again into appropriate vector DNA to transform other eukaryotic host cells. Gene expression in the host cells is enabled by further incorporating into these vectors appropriate promoters and sequences involved in the development of characteristics.

Eukaryotic host cells include cells of vertebrates, insects; and yeasts. Vertebrate cells commonly used in the art include simian COS cells (Gluzman Y., Cell 23: 175-182 (1981)), the dihydrofolate reductase deficient strain of Chinese hamster ovary cells (CHO) (Urlaub G. and Chasin, L.A., Proc. Natl. Acad. Sci. USA 77: 4216-4220 (1980)), HEK293 cells from human fetal kidney, and 293-EBNA cells (Invitrogen), which are HEK293 cells having EBNA-1 gene of Epstein Barr virus. The host cells, however, are not limited to those described above.

An expression vector for vertebrate cells may include a promoter, which is generally located upstream of the gene to be expressed, an RNA splice site, a polyadenylation site, and/or a transcription terminator sequence, and if desired, a replication origin. Exemplary non-limiting expression vectors include pSV2dhfr having SV40 early promoter (Subramani S. *et al*., Mol. Cell. Biol. 1: 854-864 (1981)), pEF-BOS having human elongation factor promoter (Mizushima S. and Nagata S. Nucleic Acids Res. 18: 5322 (1990)), and pCEP4 (Invitrogen) having cytomegalovirus promoter.

For example, when COS cells are used as host cells, suitable expression vectors may include the SV40 replication origin, which is capable of autonomous replication in COS cells, and further include a transcription promoter, a transcription-terminating signal, and an RNA splice site. Such expression vectors include pME18S (Maruyama K. and Takebe Y., Med. Immunol. 20: 27-32 (1990)), pEF-BOS (Mizushima S. and Nagata S., Nucleic Acids Res. 18: 5322 (1990)), and pCDM8 (Seed B., Nature 329: 840-842 (1987)). These expression vectors can be incorporated into COS cells by the DEAE-dextran method (Luthman H. and Magnusson G., Nucleic Acids Res. 11: 1295-1308 (1983)), the calcium phosphate-DNA co-precipitation (Graham F.L. and van der Ed A.J., Virology 52: 456-457 (1973)), the method using FuGENE6 (Boeringer Mannheim), or electroporation (Neumann E. *et al*., EMBO J. 1: 841-845 (1982)), to obtain the desired transformed cells.

When CHO cells are used as host cells, additional vectors capable of expressing the neo gene, functioning as a G418 resistant marker, may be co-transfected together with the expression vectors of the present invention. For example, pRSVneo (Sambrook, J. *et al*. (1989): "Molecular Cloning-A Laboratory Manual" Cold Spring Harbor Laboratory, NY) or pSV2-neo (Southern P.J. and Berg P., J. Mol. Appl. Genet. 1: 327-341 (1982)) may be co-transfected, and G418-resistant colonies can be selected to obtain transformed cells which stably produce the polypeptides of the present invention. When 293 cells are used as host cells, expression vectors may be co-transfected together with vectors capable of expressing a zeocin-resistant gene functioning as a zeocin-resistant marker, such as pcDNA3.1/Zeo(+) (Invitrogen); zeocin-resistant colonies can then be selected to obtain transformant cells which stably produce the polypeptides of the present invention. When 293-EBNA cells are used as host cells, an expression vector, such as pCEP4 (Invitrogen), which has replication origin of Epstein Barr virus and which is capable of autonomous replication in 293-EBNA cells can be used to obtain the desired transformant cells.

The polypeptides of the present invention can be produced by cultivating the desired transformants thus obtained. The culture medium used in such cultivation may be any medium commonly used for particular host cells employed. For example, the COS cells described above may be cultured in RPMI-1640 medium or Dulbecco's modified Eagle's minimum essential medium (DMEM), which may be supplemented with serum components, such as fetal bovine serum (FBS), if desired. For the 293-EBNA cells, culture may be performed in culture medium, such as Dulbecco's modified Eagle's minimum essential medium (DMEM), optionally containing fetal bovine serum (FBS) or other serum components, and further supplemented with G418.

The polypeptides of the present invention produced by the transformants as described above can be isolated and purified by various isolation procedures known in the art utilizing the physical and chemical properties of the polypeptides. Exemplary procedures include solubilization of the membrane fraction containing the polypeptides followed by ordinary treatment with a protein-precipitating agent, ultrafiltration, gel filtration chromatography, adsorption chromatography, ion exchange chromatography, affinity chromatography, high performance liquid chromatography (HPLC) or other liquid chromatographic process, dialysis, or combinations thereof.

The present invention relates to the thus obtained substantially pure polypeptides having AGF activity. In the present invention, the phrase "substantially pure" means, for example, that the polypeptide is free from other proteins of the source species from which the polypeptide is derived. Herein, the substantially pure polypeptide is a polypeptide having a purity of, for example, at least 80%, typically at least 90%, preferably at least 95%, and more preferably at least 99%.

When a polypeptide of the present invention is expressed after in-frame fusion with a marker sequence, confirmation of the expression of AGF and localization of AGF in cells as well as purification of AGF are enabled. Exemplary marker sequences include the FLAG epitope, the Hexa-Histidine tag, the Hemagglutinin tag, and the myc epitope. A sequence specifically recognizable by a protease, such as enterokinase, factor Xa, or thrombin, may be inserted between the marker sequence and the AGF sequence so that the marker sequence domain can be removed by cleavage using such a protease. For example, there is a report of connecting muscarinic acetylcholine receptor and Hexa-Histidine tag with the intervening thrombin-recognizing sequence (Hayashi M.K. and Haga T., J. Biochem. 120: 1232-1238 (1996)).

### 3) Tg animals manipulated to express AGF in epidermal cells:

The present invention provides Tg animals that express in epidermis a polypeptide of any one of the following (a) to (d):
(a) a polypeptide comprising the amino acid sequence of 1 to 450 of SEQ ID NO: 2 or the amino acid sequence of 1 to 433 of SEQ ID NO: 4, wherein said polypeptide has epidermal cell proliferating activity;
(b) a polypeptide comprising the amino acid sequence of 1 to 450 of SEQ ID NO: 2 or the amino acid sequence of 1 to 433 of SEQ ID NO: 4, wherein substitution, deletion, and/or insertion of one to several amino acid residues has occurred at one to several sites of said amino acid sequence, wherein said polypeptide has epidermal cell proliferating activity;
(c) a polypeptide encoded by a DNA which hybridizes with the DNA encoding the amino acid sequence of 1 to 450 of SEQ ID NO: 2 or the amino acid sequence of 1 to 433 of SEQ ID NO: 4 under stringent conditions, wherein said polypeptide has epidermal cell proliferating activity; and
(d) a polypeptide consisting of an amino acid sequence having a homology of at least 95% to the amino acid sequence of 1 to 450 of SEQ ID NO: 2 or to the amino acid sequence of 1 to 433 of SEQ ID NO: 4, wherein said polypeptide has epidermal cell proliferating activity.

The amino acid sequence set forth in SEQ ID NO: 2 is the amino acid sequence of human AGF (NL8/NEW). The nucleotide sequence of SEQ ID NO: 1 encodes the amino acid sequence of SEQ ID NO: 2. As in the case of mouse AGF, human AGF has signal sequence on its N terminal (-24 to -1) that is cleaved when the protein is secreted from cells. The human AGF without the signal sequence, namely, mature human AGF consisting of the amino acids of 1 to 450 of amino acid sequence of SEQ ID NO: 2 has the relevant physiological activity.

A polypeptide of any one of the above (a) to (d) constituting a Tg animal of the present invention is a human AGF, a mouse AGF, or an "equivalent" thereof. As confirmed in the Examples, a Tg animal manipulated to express AGF in its epidermis exhibits symptoms similar to those of psoriasis. Accordingly, the Tg animal expressing AGF or its "equivalent" in the epidermis is useful as an animal model for psoriasis.

In the present invention, "expression of a polypeptide in the epidermis" includes not only the expression that specifically takes place in the epidermis but also the case further associated with the expression in tissues apart from the epidermis. The Tg mice of the present invention can be produced by a method commonly used in producing Tg animals (see, *e*.*g*., "Current Manual for Animal Cell Experiments" LIC, Chapter 7, pages 361 to 408 (1990)).

More specifically, for example, the TG mice of the. present invention can be produced by the procedure described in Example 8. In another method, using ES cells, ES cells lacking the hypoxanthine-guanine phosphoribosyltransferase gene (HRPT negative) derived from normal mouse blastocysts are fused with yeast harboring a YAC vector comprising a gene encoding an AGF of the present invention or a part thereof and an HRPT gene by spheroplast fusion method. The ES cells carrying the exogenous genes integrated into mouse endogenous genes are selected by HAT selection. Next, the selected ES cells are introduced into fertilized eggs (blastocysts) obtained from normal mice by microinjection (Proc. Natl. Acad. Sci. USA 77(12): 7380-7384 (1980); USP 4,873,191). These blastocysts are transplanted into the uterus of different normal mice, and these surrogate mother mice produce chimeric Tg mice. These chimeric Tg mice are mated with normal mice to produce heterozygous Tg mice. The heterozygous Tg mice thus obtained are then mated to produce homozygous Tg mice according to Mendel's law.

Other methods that can be used to produce the Tg animals of the present invention include known methods, such as the method in' which genes and eggs are mixed and treated with calcium phosphate without using ES cells; the method in which genes are directly introduced into the nucleus of eggs at the pronucleus stage with micropipettes under a phase contrast microscope (Microinjection, USP 4,873,191); the method in which genes are inserted into retrovirus vectors to infect eggs; and the sperm vector method in which genes are introduced into eggs by sperms (M. Lavitrano *et al*., Cell 57(5): 717-723 (1989)).

The Tg animals of the present invention manipulated to express AGF in the epidermis can be produced by using any vertebrates other than humans. For example, Tg animals having various introduced genes or altered expression levels have been produced from vertebrates such as mice, rats, rabbits, minipigs, goats, sheep, and bovines. The Tg animals of the present invention can also be produced using animals including such species.

The Tg animals of the present invention exhibit significant thickening of the epidermal cell layer as well as enhanced proliferative activity in the thickened epidermal cells. Since such phenotypes resemble the symptoms of the psoriasis patients, these animals are useful as model animals for skin diseases. Exemplary skin diseases include psoriasis, allergic diseases, urticaria, edema, and inflammation. Accordingly, the Tg animals of the present invention can be used not only in the screening of pharmaceuticals used for treating and preventing diseases such as psoriasis, allergic diseases, urticaria, edema, and inflammation, but also in elucidating the pathological mechanisms of such diseases and for the safety test of the pharmaceuticals screened.

### 4) Psoriasis model animals:

Based on the findings of the Tg animals described above, the present invention provides an animal model for psoriasis comprising Tg animals manipulated to express AGF in the epidermis. In other words, this invention relates to psoriasis model animals comprising Tg animals manipulated to express in the epidermis a polypeptide of any one of the following (a) to (d). The present invention also relates to methods for producing psoriasis model animals, comprising the step of manipulating the overexpression of a polypeptide of any one of the following (a) to (d) in epidermis of non-human animals:
(a) a polypeptide comprising the amino acid sequence of 1 to 450 of SEQ ID NO: 2 or the amino acid sequence of 1 to 433 of SEQ ID NO: 4, wherein said polypeptide comprises epidermal cell proliferating activity;
(b) a polypeptide comprising the amino acid sequence of 1 to 450 of SEQ ID NO: 2 or the amino acid sequence of 1 to 433 of SEQ ID NO: 4, wherein substitution, deletion, and/or insertion of one to several amino acid residues has occurred at one to several sites of said amino acid sequence, wherein said polypeptide has epidermal cell proliferating activity;
(c) a polypeptide encoded by a DNA which hybridizes with the DNA encoding the amino acid sequence of 1 to 450 of SEQ ID NO: 2 or the amino acid sequence of 1 to 433 of SEQ ID NO: 4 under stringent conditions, wherein said polypeptide has epidermal cell proliferating activity; and
(d) a polypeptide consisting of an amino acid sequence having a homology of at least 95% to the amino acid sequence of 1 to 450 of SEQ ID NO: 2 or to the amino acid sequence of 1 to 433 of SEQ ID NO: 4, wherein said polypeptide has epidermal cell proliferating activity.

The amino acid sequence set forth in 1 to 450 of SEQ ID NO: 2 is encoded by the nucleotide sequence of 61 to 1410 of SEQ ID NO: 1. The cDNA comprising the nucleotide sequence of 1 to 1410 of SEQ ID NO: 1 is known as human NL8/NEW. The amino acid sequence of 1 to 450 of SEQ ID NO: 2 (human) has a homology of 76% to the amino acid sequence of 1 to 433 of SEQ ID NO: 4 (mouse). In particular, the fibrinogen domain on the C terminal side, which is known to play an important role in the activity of proteins belonging to angiopoietin family (William N. Procopio *et al*., J. Biol. Chem. 274: 30196-30201 (1999)), has a homology as high as 89%. Such a high homology in the area significant to maintaining the activity suggests that human AGF has an activity equivalent to that of the mouse AGF.

As described above, the Tg animals manipulated to express mouse AGF in the epidermis exhibit phenotypes resembling the skin symptoms of the psoriasis patients. Accordingly, the Tg animals manipulated to express in the epidermis human AGF, which is expected to have a function similar to that of the mouse AGF, or a protein which is functionally equivalent to the human or the mouse AGF should also be useful as animal models for psoriasis.

The polynucleotide consisting of the nucleotide sequence of 61 to 1410 of SEQ ID NO: 1 or 73 to 1371 of SEQ ID NO: 3 used in the present invention or an equivalent thereof can be obtained by the same procedure as the polynucleotide of the present invention, based on their nucleotide sequence information.

The psoriasis model animals of the present invention can be used in the screening of candidate substances useful for treating psoriasis. This screening method comprises, for example, the following steps:
i) administering a candidate substance to the psoriasis model animal manipulated to express' the polynucleotide of any one of the (a) to (d) in the epidermis;
ii) monitoring the state of the epidermal tissue in the psoriasis model animal; and
iii) selecting the candidate substance that has the action of normalizing the epidermal tissue as compared with a control.

In the screening method described above, the "normalization" of the epidermal tissue refers to alleviating the phenotypes unique to the psoriasis model animal of the present invention. Normalization of the epidermal tissue can be detected by using as an index, for example, thickening of the epidermal tissue or the proliferative activity of epidermal cells. The candidate substance is determined to have therapeutic effects for psoriasis when such an index resembles that of wild-type animals as compared to a control. In the screening method described above, a Tg animal which has not received the candidate substance can be used as a control. In addition, using a Tg animal which has received a substance which clearly has therapeutic effects for psoriasis as the control, substances can be selected for therapeutic effects equivalent to or greater than that of the substance.

### 5) Tissue regenerative agents of the present invention:

The present invention includes tissue regenerative agents which contain AGF or a polynucleotide encoding AGF as its active ingredient. A polypeptide of any one of the following (a) to (d) can be used as AGF in a tissue regenerative agent of this invention:
(a) a polypeptide comprising the amino acid sequence of 1 to 450 of SEQ ID NO: 2 or the amino acid sequence of 1 to 433 of SEQ ID NO: 4, wherein said polypeptide has epidermal cell proliferating activity;
(b) a polypeptide comprising the amino acid sequence of 1 to 450 of SEQ ID NO: 2 or the amino acid sequence of 1 to 433 of SEQ ID NO: 4, wherein substitution, deletion, and/or insertion of one to several amino acid residues has occurred at one to several sites of said amino acid sequence, wherein said polypeptide has epidermal cell proliferating activity;
(c) a polypeptide encoded by a DNA which hybridizes with the DNA encoding the amino acid sequence of 1 to 450 of SEQ ID NO: 2 or the amino acid sequence of 1 to 433 of SEQ ID NO: 4 under stringent conditions, wherein said polypeptide has epidermal cell proliferating activity; and
(d) a polypeptide consisting of an amino acid sequence having a homology of at least 95% to the amino acid sequence of 1 to 450 of SEQ ID NO: 2 or to the amino acid sequence of 1 to 433 of SEQ ID NO: 4, wherein said polypeptide has epidermal cell proliferating activity.
   The polypeptide which is the active ingredient of the tissue regenerative agent of the present invention is preferably the polypeptide consisting of the amino acid sequence of 1 to 450 of SEQ ID NO: 2 or the polypeptide consisting of the amino acid sequence of 1 to 433 of SEQ ID NO: 4.
   In addition, the present invention relates to a tissue regenerative agent containing as its active ingredient a polynucleotide of any one of the following (e) to (h):
(e) a polynucleotide comprising the nucleotide sequence of 61 to 1410 of SEQ ID NO: 1 or the nucleotide sequence of 73 to 1371 of SEQ ID NO: 3, wherein said polynucleotide encodes a polypeptide having epidermal cell proliferating activity;
(f) a polynucleotide encoding a polypeptide comprising the amino acid sequence of 1 to 450 of SEQ ID NO: 2 or the amino acid sequence of 1 to 433 of SEQ ID NO: 4 wherein substitution, deletion, and/or insertion of one to several amino acid residues has occurred at one to several sites of said amino acid sequence, wherein said polypeptide has epidermal cell proliferating activity;
(g) a polynucleotide which hybridizes with the DNA shown in the nucleotide sequence of 61 to 1410 of SEQ ID NO: 1 or the nucleotide sequence of 73 to 1371 of SEQ ID NO: 3 under stringent conditions, wherein said polynucleotide encodes a polypeptide having epidermal cell proliferating activity; and
(h) a polynucleotide encoding a polypeptide consisting of an amino acid sequence having a homology of at least 95% to the amino acid sequence of 1 to 450 of SEQ ID NO: 2 or to the amino acid sequence of 1 to 433 of SEQ ID NO: 4, wherein said polypeptide has epidermal cell proliferating activity.

The polynucleotide which is the active ingredient in the tissue regenerative agent of the present invention is preferably a polynucleotide consisting of the nucleotide sequence of 61 to 1410 of the nucleotide sequence of SEQ ID NO: 1 or the nucleotide sequence of 73 to 1371 of SEQ ID NO: 3.

In the present invention, the term "tissue regenerative agent" refers to a drug which promotes the regeneration of a tissue which has been injured by any means. Such injuries of tissues include conditions exhibiting injury, loss, degeneration and loss, and fibrosis of tissues. The cause of the injury, loss, degeneration and loss, and fibrosis is not limited. The term "tissue regeneration" refers to proliferation of the cells constituting the tissue and reconstitution of the tissue having the normal function. In the present invention, the term "reconstitution" means that the tissue is restored to a state which medically shows the achievement of therapeutic effects. Thus, it also includes incomplete restoration of the function of the original tissue. For example, in the reconstitution of skin, therapeutic effects can medically be achieved when the continuum of epidermal or dermal tissue has been restored, and in general, complete restoration of the skin color, sweat gland, hair root, nerve, and the like is not necessarily required. The present invention, of course, enables regeneration to a higher degree as well. The regeneration action can be evaluated by confirming the faster regeneration enabled by the treatment according to the present invention.

In the present invention, tissue regeneration includes not only enhancement of the regenerative function inherent to the tissue to be regenerated, but also the regeneration of a tissue which is substantially free from the regenerative function. Tissues such as skin, liver, muscle, and blood vessels have the function of regenerating themselves, so long as the damaged area is small. On the other hand, cartilage is not expected to undergo self-regeneration. Nevertheless, the present invention enables tissue regeneration even if the damaged area is large or even in the tissue with no self-regenerative function.

Exemplary tissues that may be regenerated by the present invention include skin tissues, epithelial tissues, cartilage tissues, and connective tissues. The epithelial tissues include epithelium of the digestive tract and epidermal tissue. The skin tissues include epidermal tissue, dermal tissue, and subcutaneous tissue. The connective tissues include subcutaneous tissue and cartilage tissue. Exemplary tissue regenerative agents include wound healing agents (healing agents for the excoriation, avulsion, incision wound, septic wound, perforating wound, burn wound, frostbite, ulcer, UV injury, radiation damage, electric injury, surgical wound, vesicular skin disease, decubitus, etc.), osteoarthritis healing agents, mucosal tissue regenerative agents, and liver regenerative agents. The polypeptides of the present invention according to the above (a) to (d) preferably have regenerative activity for all of the skin tissue, epithelial tissue, cartilage tissue, and connective tissue. It is the new finding discovered by the present inventors that the polypeptide according to the above (a) to (d) has regenerative activity for the cells constituting such tissues.

The tissue regenerative agents of the present invention are useful as wound healing agents or as agents for promoting wound healing. More specifically, the present invention provides a wound healing agent comprising a polypeptide according to any one of the above (a) to (d) as its active ingredient. The present invention also provides a wound healing agent comprising a polynucleotide according to any one of the above (e) to (h) as its active ingredient.

In the present invention, the term "wound" designates injury, loss, and/or degeneration and slough of the epidermal tissue, and the cause of the injury, loss, and/or degeneration and slough is not particularly limited. Exemplary wounds include excoriation, avulsion, incision wounds, septic wounds, perforating wounds, burn wounds, frostbite, ulcers, UV injury, radiation damage, electric injury, surgical wounds, vesicular skin disease, and decubitus. Healing of the wound involves restoration of the continuum of the tissue which lost the continuum by injury. The enhancement of healing refers to the enhancement of the healing capability inherent to an organism. Enhancement of the healing capability can be evaluated by confirming the faster healing by the treatment according to the present invention.

The present invention provides a therapeutic agent for osteoarthritis comprising a polypeptide according to any one of the above (a) to (d) or a polynucleotide according to any one of the above (e) to (h) as its active ingredient. Osteoarthritis is a disease associated with the loss and/or degeneration of joint cartilage tissue. The therapeutic effects for osteoarthritis can be evaluated by confirming that the cartilage tissue has been regenerated and that the loss and/or degeneration of joint cartilage tissue has been controlled by the treatment according to the present invention. The present invention also relates to a method of tissue regeneration comprising the step of administering a polypeptide according to any one of the above (a) to (d) or a polynucleotide according to any one of the above (e) to (h). Further, the present invention relates to use of a polypeptide according to any one of the above (a) to (d) or a polynucleotide according to any one of the above (e) to (h) in the production of the tissue regenerative agent.

Furthermore, the present invention relates to a method of wound healing comprising the step of administering a polypeptide according to any one of the above (a) to (d) or a polynucleotide according to any one of the above (e) to (h). The present invention also relates to a method for promoting wound healing comprising the step of administering a polypeptide according to any one of the above (a) to (d) or a polynucleotide according to any one of the above (e) to (h). Further, the present invention relates to use of a polypeptide according to any one of the above (a) to (d) or a polynucleotide according to any one of the above (e) to (h) in the production of the wound healing agent.

Furthermore, the present invention relates to a method for treating osteoarthritis comprising the step of administering a polypeptide according to any one of the above (a) to (d) or a polynucleotide according to any one of the above (e) to (h). In addition, the present invention relates to use of a polypeptide according to any one of the above (a) to (d) or a polynucleotide according to any one of the above (e) to (h) in the production of the therapeutic agent for osteoarthritis.

### 6) Administration of a wound healing agent, tissue regenerative agent, or therapeutic agent for osteoarthritis of the present invention:

A wound healing agent or tissue regenerative agent of the present invention may be administered orally in the form of, for example, a tablet, pill, capsule, granule, fine granule, powder, or peroral solution, or alternatively, parenterally , in the form of an intravenous injection, intramuscular injection, joint injection, or other injections, suppository, percutaneous dosage form, or permucosal dosage form. In particular, in the case of the polypeptide which is influenced by an enzyme, it is preferably administered by percutaneous administration, intravenous injection, or other parenteral administration, or alternatively, by using a drug delivery technology that enables delivery of the polypeptide without decomposition to the lower part of the digestive tract (for example, jejunum, ileum, colon, or large intestine), where effects of digestive enzymes are relatively small.

A preparation comprising a polypeptide of any one of the above (a) to (d) as its active ingredient can be prepared as a pharmaceutical composition by using a pharmaceutically acceptable carrier, excipient, or other additives which are commonly used in the preparation of a polypeptide into a pharmaceutical composition.

Injection for parenteral administration may be in the form of a sterile aqueous or non-aqueous solution, suspension, or as an emulsion. An aqueous solution or suspension contains, for example, a diluent, such as distilled water for injection and physiological saline. A non-aqueous solution or suspension may contain a diluent, for example, propylene glycol, polyethyleneglycol, vegetable oil such as olive oil, alcohol such as ethanol, or polysorbate 80. The composition may further contain a humectant, an emulsifier, a dispersant, a stabilizer, a solubilizer or a solubilization aid, a preservative, and/or the like. The composition may be sterilized, for example, by filtration through a bacterial filter, by incorporating a bacteriocide, or by irradiation. A sterile solid composition can be produced and dissolved in sterile water or another sterile injection medium before use.

A therapeutic agent for treating a skin wound of the present invention may take any dosage form, and dosage forms for topical preparation include ointments, creams, gels, gel ointments, tapes, solutions, and powders. Such preparations can be produced by a method commonly used in the art using ordinary excipients, diluents, carriers, and the like.

For example, petrolatum, higher alcohols, beeswax, vegetable oils, and polyethyleneglycol, may be employed in the preparation of ointments. Use of a commercially available ointment base, such as "plastibase", is also convenient. Oils, waxes, higher fatty acids, higher alcohols, fatty acid esters, purified water, emulsifiers, and the like, may be employed in the preparation of creams. Sodium polyacrylate or the like, hydroxypropylcellulose, hydroxypropylmethylcellulose, polyvinyl alcohols, polyvinylpyrrolidone, purified water, lower alcohols, polyhydric alcohols, polyethyleneglycol, carboxyvinyl polymer, and the like, may be employed in the preparation of gels. The base that is used for the gel, an emulsifier (preferably a nonionic surfactant), and an oil (such as liquid paraffin) may be employed in the preparation of a gel ointment. The topical preparation may further include paraffin, squalane, lanolin, cholesterol ester, and'the like, and optionally an antioxidant, such as BHA and BHT.

The wound healing agents, tissue regenerative agents, and therapeutic agents for osteoarthritis of the present invention comprise AGF or its equivalent at an amount sufficient for realizing the intended effects in the wound treatment or prevention or in the tissue regeneration, namely, a therapeutically effective amount or a pharmacologically effective amount of AGF or its equivalent. The effective dose in actual administration can be confirmed, for example, by measuring the extent of recovery or regeneration of the target disease. The actual dose to be administered depends on the symptom, age, weight, and other factors of the individual being treated, and the dose is preferably determined so that the desired effects are realized without inducing any significant side effects. Those skilled in the art can readily determine such an effective dose and toxicity by using model animals known in the art.

A polynucleotide encoding AGF can be administered instead of AGF protein in a gene therapy as the wound healing agent, tissue regenerative agent, or therapeutic agent for osteoarthritis. Administration methods used in gene therapies are broadly classified into those using non-viral vectors and those using viral vectors ("Jikkenigaku (Experimental Medicine), Special Edition, Basic Technologies of Gene Therapy" Yodosha (1996); "Jikkenigaku (Experimental Medicine), Special Edition, Experimental Methods of Gene Transfer and Expression Analysis" Yodosha (1997); The Japan Society of Gene Therapy Ed. "Handbook on Research and Development of Gene Therapy" NTS (1999)).

Exemplary methods of gene introduction into tissue using a non-viral vector include those using an encapsulating liposome; those using an electrostatic liposome; those using a HVJ-liposome method (for example, J. Clin. Invest. 93: 1458-1464 (1994)); those utilizing receptor-mediated introduction; those in which the gene is introduced into the cell with a carrier, such as a metal particle, by using a particle gun; those in which a plasmid is directly introduced; and those in which the gene is introduced by a positively charged polymer. Exemplary known methods of introducing a gene into a cell include methods using lipofection, calcium phosphate co-precipitation, DEAE-dextran, and direct injection of DNA using glass microtube.

Known viral vectors include retrovirus vectors, adenovirus vectors, and adeno-associated virus vectors. More specifically, gene introduction may be accomplished, for example, by introducing a gene encoding an AGF into a DNA or RNA virus, such as detoxicated retrovirus, adenovirus, adeno-associated virus, herpesvirus, vaccinia virus, poxvirus, poliovirus, Sindbis virus, SV40, HIV, or Sendai virus, and infecting the cell with such virus.

The wound healing agent, the tissue regenerative agent, and the therapeutic agent for osteoarthritis comprising a gene of the present invention can be administered either by an in vivo method, in which the gene is directly introduced into the body, or by an ex vivo method, in which the gene is introduced into cells taken out of the body and the cells having the introduced gene are returned to the body.

Dosage forms used for the in vivo administration include solutions adapted for use in injection and infusion. More specifically, the solution can be prepared, for example, by dissolution in a solvent such as PBS or other buffer solution, physiological saline, or sterilized water followed by optional sterilization by filtration or the like and filling in a sterile container. If necessary, the solution can be prepared in suspended, frozen, centrifuged/concentrated/frozen, or other form. The gene of the present invention can also be topically applied to the lesion in the form of a semisolid ointment containing a fat, fatty oil, lanolin, petrolatum, wax, liquid paraffin, plaster, plastics, glycol, higher alcohol, resin, glycerin, emulsifier, and/or suspending agent. If desired, such an ointment can be formed into a sheet by mixing with a hydrophilic polymer to adhere to the lesion.

The wound healing agent, the tissue regenerative agent, and the therapeutic agent for osteoarthritis including the gene of the present invention can be administered by selecting a suitable method and site depending on the type and symptom of the disease. The administration method is preferably parenteral administration, and in particular, administration to the wounded site. More specifically, the agent can be administered into the blood vessel, muscle, or articular cavity, or to the surface by an ointment or the like. Administration into the blood vessel, muscle, articular cavity, and the like may be accomplished by such means as injection and catheterization. The agent can be administered to the wound surface in the form of an ointment or the like, thereby promoting the proliferation of the epidermal cell and regeneration of the epidermal tissues of the lesion for recovery and normalization of the function of the lesion.

The healing agent of the present invention comprises a polynucleotide encoding AGF or its equivalent at an amount sufficient for realizing the intended effects in the wound treatment or prevention, namely, a therapeutically effective amount or a pharmacologically effective amount of AGF or its equivalent. Confirmation of the effective dose in actual administration may be accomplished, for example, by measuring the extent of the target disease recovery. The actual dose to be administered depends on the symptom, age, weight, and other factors of the individual to be treated, and the dose is preferably determined so that the desired effects are realized without inducing any significant side effect. Those skilled in the art can readily determine effective dose, toxicity, and such, by using wound model animals known in the art. For example, the typical dose of active ingredient for an adult is preferably 0.1 µg to 200 mg per day, and more preferably, 0.1 µg to 20 mg per day.

The wound healing agent and the tissue regenerative agent of the present invention can optionally contain known factors that are conventionally used in the wound healing or tissue regeneration in addition to AGF or the polynucleotide encoding the AGF. Such factors include KGF-1, KGF-2, basic FGF, acidic FGF, PDGF, EGF, TGF-α, TGF-β, NDF, and IGF-1.

### Brief Description of the Drawings

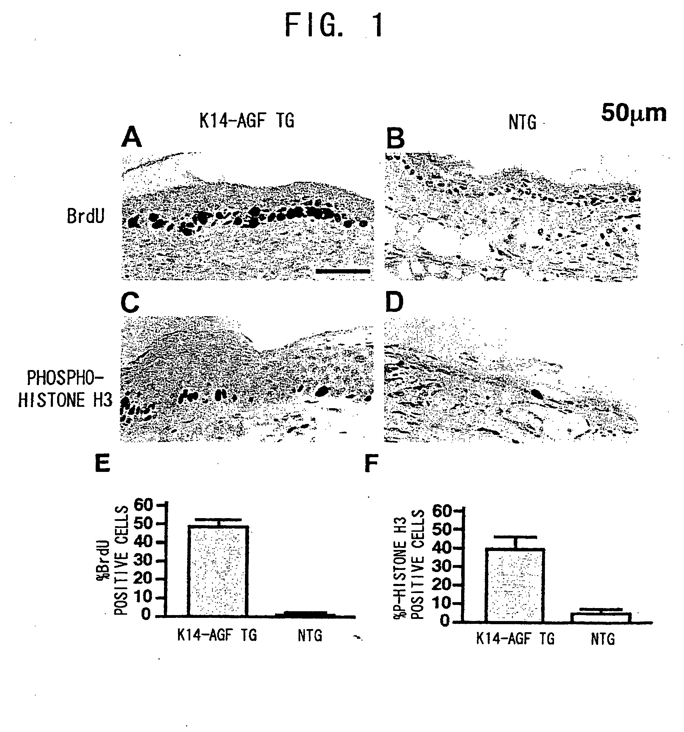

FIG. 1 is a series of photographs depicting the results when the proliferative activity and the DNA synthesizing ability of the epidermal cells of an AGF transgenic mouse were studied.

A and B depict the results of incorporation of BrdU in the epidermal cells. C and D depict the results when DNA synthesizing ability of epidermal cells was studied by the staining using the anti-phospho-Histone H3 antibody. A and C depict the results when the tissue from the transgenic mouse was used, and B and D depict the results when the tissue was from a control mouse, having no AGF gene introduced. E and F are graphs depicting the results of a cell counting assay wherein cells stained by BrdU (E) and anti P-Histone H3 (F).

FIG. 2 is a series of photomicrographs depicting blood vessels visualized with FITC. A: normal mouse, B: transgenic mouse.

FIG. 3 is a series of photographs depicting the results when closure of the ear punch hole was studied in the AGF Tg mouse. The cartilage tissue in I and J is indicated by the arrowhead.

A and B: size of the ear punch hole in the AGF Tg mouse.

C and D: size of the ear punch hole in the normal mouse.

E: thin section of the tissue from the area with no ear punch hole.

F, G, H: thin section of the tissue from the area where tissue regenerated after punching the hole.

I: magnified view of an area in F where no punch hole was formed.

J: magnified view of another area in F where tissue regenerated after punching the hole.

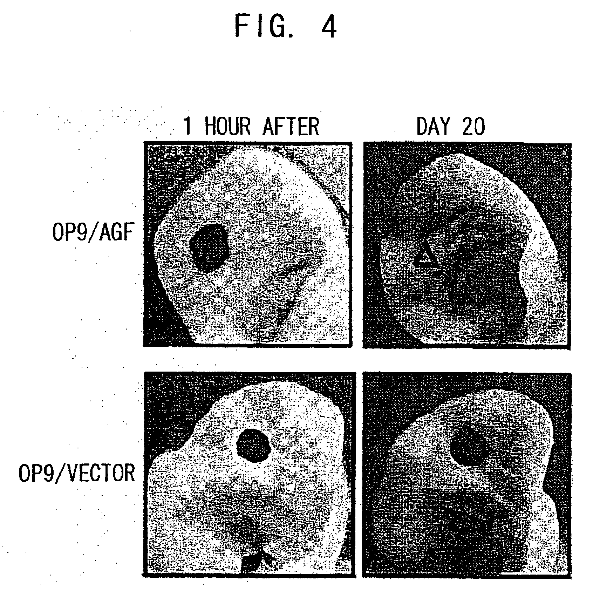

FIG. 4 is a series of photographs demonstrating the activity of closing the ear punch hole of the recombinant AGF from OP9 cell. Upper photographs depict changes in the size of the ear punch hole upon administration of the cell line stably expressing the mouse AGF (OP9/AGF). Lower photographs depict changes in the size of the ear punch hole upon administration of the OP9 cell line (OP9/vector) transfected with the vector alone.

### Best Mode for Carrying out the Invention

The present invention is described in detail below, but is not to be construed as being limited thereto. Unless otherwise noted, the procedures were performed according to known methods (for example, Sambrook J. *et al*. "Molecular Cloning-A Laboratory Manual", Cold Spring Harbor Laboratory, NY, (1989)).

### [Example 1] Cloning of mouse AGF

In amplifying the novel angiopoietin-related growth factor AGF, SEQ ID NO: 5 was used as the forward primer, and SEQ ID NO: 6 was used as the reverse primer. RT-PCR was conducted using Pyrobest DNA polymerase (Takara Shuzo), and repeating the cycle of 98°C (20 seconds)/64°C (30 seconds)/74°C (3 minutes) 35 times in the presence of 5% formamide to amplify the DNA fragment of about 1.5 kbp. This fragment was cloned using pCR2.1 plasmid (Invitrogen) to obtain the plasmid pCR2.1-mNew. The resulting clone was analyzed for the nucleotide sequence by the dideoxy termination method using ABI377 DNA Sequencer (Applied Biosystems). The sequence found is shown in SEQ ID NO: 3. This sequence has an open reading frame of 1374 bases (1 to 1374 in SEQ ID NO: 3). The amino acid sequence (457 amino acids) deduced from the open reading frame is shown in SEQ ID NO: 4.

### [Example 2] Distribution analysis of the human AGF gene expression

Expression of mRNA was investigated using a Northern blot hybridization.assay with the mRNA. Hybridization was conducted using membranes of Human MTN Blot, Human MTN Blot II, Human MTN Blot III, and Human MTN Blot Fetal II from Clontech. The probe used was prepared using as the source the human AGF gene of 1.4 kb having the nucleotide sequence of SEQ ID NO: 1, and using a DNA labeling kit (BcaBEST DNA labelling system: Takara). The hybridization solution was 50% formamide, 5x SSPE, 5x denhardt (0.1% Ficoll, 0.1% polyvinylpyrrolidone, 0.1% BSA), 0.5% SDS, and heat-denatured salmon sperm DNA (200 µg/mL). Prehybridization was conducted at 42°C for 2 hours, and hybridization was conducted at 42°C for 16 hours. The membrane was washed in 2x SSPE/0.1% SDS at room temperature for 10 minutes, in 1x SSPE/0.1% SDS at 65°C for 15 minutes, and in 0.5x SSPE/0.1% SDS at 65°C for 15 minutes, and after air drying, autoradiographed using an intensifying screen. As a result, mRNA with the size of about 2.0 kb was specifically detected in adult and fetal liver at high levels, but not detected in other organs.

### [Example 3] Distribution analysis of the mouse AGF gene expression

Expression of mRNA was investigated using a Northern blot hybridization assay with the mRNA. The hybridization was conducted using membranes of Mouse MTN Blot and Mouse MTN Blot embryo from Clontech. The probe used was prepared using as the source the mouse AGF gene of 1.4 kb obtained in the above cloning, and using a DNA labeling kit (BcaBEST DNA labelling system: Takara). The hybridization solution was the ExpressHyb Hybridization Solution (Clontech). Prehybridization was conducted at 42°C for 2 hours, and hybridization was conducted at 42°C for 16 hours. The membrane was washed in 2x SSPE/0.1% SDS at room temperature for 10 minutes, in 1x SSPE/0.1% SDS at 68°C for 15 minutes, and in 0.5x SSPE/0.1% SDS at 68°C for 15 minutes, and after air drying, autoradiographed using an intensifying screen. As a result, mRNA with the size of about 4.2 kb and 2.0 kb was detected at high levels in adult liver; about 4.2 kb,.2.0 kb, and 1.2 kb in day 15 and day 18 embryos; about 4.2 kb in day 7 embryos; about 2.0 kb and about 1.2 kb in day 11 embryos; while it was hardly detected in other organs.

### [Example 4] Production of an AGF-expressing OP9 cell line

The pCR2.1-mNew produced in Example 1 was cleaved with restriction enzymes XbaI and SpeI to produce a fragment comprising the mouse AGF gene with the size of 1.4 kb. This fragment was inserted into pEF-BOS-neo (Mizushima, S., & Nagata, S. Nucleic Acids Res. 18: 5322 (1990)) which had been cleaved with XbaI and treated with BPA, to produce an expression vector for the mouse AGF, pEF-BOS-mAGF. pEF-BOS-mAGF was introduced into OP9 cells using the Fugene6 method (Roche Diagnostics) according to the manufacture's protocol. A cell line (OP9/AGF) stably expressing mouse AGF was obtained by cultivating the transfected cells in the presence of 300 µg/ml Geneticin (Roche Diagnostics). An OP9 cell line (OP9/vector) transfected with pEF-BOS-neo vector comprising no mouse AGF gene was also produced for use as a negative control.

### [Example 5] Production of anti-mouse AGF antibody

An antibody specifically recognizing mouse AGF was prepared by a method commonly used in the art, namely by immunizing a mouse five times with a conjugate of a synthetic peptide comprising the amino acid sequence of SEQ ID NO: 7 and KLH. The resulting antibody was purified using a MabTrap Kit (Amersham Pharmacia) according to the manufacture's protocol to obtain IgG.

The activity of the resulting antibody to bind to the AGF protein was confirmed by Western blotting using the recombinant AGF protein. The AGF-expressing OP9 cell line produced in Example 4 was cultivated overnight in a serum-free culture medium, and the culture supernatant was subjected to polyacrylamide gel electrophoresis and transferred to a PVDF membrane using a blotting device. After the transfer, the PVDF membrane was blocked by adding 10 mM Tris-HCl, 0.15 M NaCl, pH 7.4, 0.05% Tween-20, and 1% BSA (blocking agent). The membrane was then reacted with the anti AGF antibody prepared as described above, and subsequently, with horseradish peroxidase-labeled goat anti-rabbit IgG polyclonal antibody (Biosource). After the reaction, binding activity of the antibody to the mouse AGF protein was confirmed using an ECL Western blotting detection 'system (Amersham Pharmacia). A band of about 50 kDa which reacted with the recombinant mouse AGF protein expressed in the OP9 cells was detected, and this molecular weight was consistent with the predicted molecular weight for the mouse AGF protein.

### [Example 6] Immunostaining of AGF protein

As the Northern blot results demonstrate, the mouse AGF gene is specifically expressed in the liver tissues. Expression of the AGF protein in the skin of a normal mouse was studied using the prepared antibody recognizing the mouse AGF. When the tissue sections of the normal mouse ear were stained with the anti-mouse AGF antibody, particular cells in the dermis and the subcutaneous tissue were stained while other cells, including the epidermal cells, were not stained at all. In view of the cell morphology and presence of many granules in the cytoplasm, the cells stained by the anti-AGF antibody were estimated to be mast cells. To confirm this estimation, serial sections of the tissue stained by the anti-mouse AGF antibody were further stained with toluidine blue, which stains granules of mast cells. It was then found that the same cells as those stained by the mouse AGF antibody were stained with the toluidine blue, indicating that these cells were the mast cells. Furthermore, granules were found to be specifically stained. These results indicate that, in the normal skin tissue, the mouse AGF is present in the granules of the mast cells.

### [Example 7] Secretion of AGF protein from mast cells

The immunohistological staining in Example 6 demonstrated that the AGF protein is present in the granules of the mast cells. It has been reported that, in various allergic diseases and inflammatory diseases, release of various mediators from the granules of the activated mast cells resulted in the pathological conditions and deterioration. Degranulation of the mast cells is also known to be involved in the wound healing. It was thus expected that, if AGF was involved in such diseases or wound healing, the AGF protein would be released by the stimulus of the mast cells. This expectation was confirmed by the procedure described below. Bone marrow was collected from an 8 week old male C57BL/6 mouse, and the collected bone marrow was cultivated in DMEM supplemented with 10% fetal bovine serum, β-mercaptoethanol, and interleukin-3 (10⁻⁵ mole/1). The medium was changed every three days, and the cultivation was continued for 8 weeks to obtain a culture of mouse mast cells. The mast cells thus obtained were stimulated with Phorbol 12-myristate 13-acetate (PMA) or with the antigen and IgE to cause degranulation. The release of the AGF by the degranulation of the mast cells was confirmed by Western blotting by detecting the AGF protein released to the reaction solution.

The reaction solution and the mast cells before the stimulus and one hour after the stimulus were subjected to SDS polyacrylamide electrophoresis, and after the electrophoresis, transferred to a PVDF membrane. After the transfer, the PVDF membrane was blocked, and the membrane was reacted with the anti-mouse AGF antibody prepared in Example 6, and then, with horseradish peroxidase-labeled rabbit anti-rabbit IgG polyclonal antibody (Biosource). After the reaction, the presence of the AGF in the reaction solution and in the cells was confirmed using an ECL Western blotting detection system (Amersham Pharmacia). As a result, while no AGF band was detected in the reaction solution before the stimulation, the AGF protein band of 50 kDa was detected in the reaction solution after the stimulation. In the mast cells, concentration of the AGF protein band of 50 kDa after the stimulus was lower than the concentration before the stimulus. These findings confirmed that the AGF is released by the degranulation of the mast cells.

### [Example 8] Production of AGF Tg mouse

An AGF Tg mouse manipulated to express mouse AGF in the epidermal cells was produced by the procedure described below.

Human K14 Promoter (2 kb), rabbit β globin intron (0.8 kb), and human K14 polyA signal (0.5 kb) were inserted into the multicloning site of pBSII KS(+) to prepare hK14PIMApBSIIKS(+) (T. Kunisada *et al*., Mech. Develop. 94 (1-2) : 67-78 (2000)). This hK14PIMApBSIIKS(+) was cleaved with restriction enzyme KpnI, and after treating with BAP, the double stranded oligonucleotide prepared by annealing the nucleic acid represented by SEQ ID NO: 8 and the nucleic acid represented by SEQ ID NO: 9 was inserted. SalI site was then inserted into the 5' side of the human K14 Promoter to prepare plasmid hK14PIMApBSIIKS+SalI.

The pCR2.1-mNew prepared in Example 1 was cleaved with restriction enzymes XbaI and SpeI to obtain the fragment comprising the mouse AGF gene of 1.4 kb. This fragment was inserted into hK14PIMApBSIIKS+SalI which had been cleaved with XbaI and treated with BPA, to produce a plasmid comprising the mouse AGF gene between a rabbit β globin intron and a human K14 poly A signal. This plasmid was cleaved with SalI, isolated, and purified to obtain K14-mAGF, which is a fragment of about 4.7 kb in which the K14 promoter, rabbit β globin intron, mouse AGF, and human K14 poly A signal are arranged in this order.

K14-mAGF was microinjected into 250 fertilized eggs of F1 hybrid mice between C57BL/6 mice and DBA2 mice, and these fertilized eggs were transplanted into the fallopian tube of the surrogate mother ICR mice (Hogan, B. *et al*. (1986). Manipulating the mouse embryo: a laboratory manual, Plainview, New York: Cold Harbor Press). The pregnant mice were allowed to have a natural birth, and the resulting 88 baby mice were evaluated whether they were Tg mice.

In order to identify the Tg mice, PCR was conducted using the genomic DNA isolated from the tail of the pup as the template. The tail was treated with Proteinase K, and the DNA was extracted with phenol/chloroform. The extracted DNA was collected by precipitation in isopropanol and precipitation in ethanol, and the precipitate was dissolved in a TE solution. The primers comprising the following nucleotide sequences were designed on the basis of the sequence of β globin intron and the cDNA sequence of the mouse AGF (SEQ ID NO: 3) in the K14-mAGF.
Forward primer: SEQ ID NO: 10
Reverse primer: SEQ ID NO: 11

Using PCR with these primers, a fragment of 760 bp was expected to be amplified from the introduced gene but not from the mouse genomic DNA. PCR was conducted using the above primers for the genomic DNA of the pup obtained. In the PCR, DNA polymerase (AmpliTaq, Roche) was used. After conducting the thermal denaturing at 95°C (5 minutes), 30 cycles of 95°C (1 minute), 60°C (1 minute), and 72°C (1 minute), and the elongation reaction at 72°C (7 minutes), the size of the amplified fragment was evaluated. 16 pups out of 88 pups were then identified'as the Tg mice.

In order to confirm that the introduced gene was actually functioning and that AGF mRNA was excessively expressed, expression of AGF mRNA in the skin of Tg mice was analyzed. 16 Tg mice were mated with Balb/c mice in order to produce the F1 mice for use in the analysis of mRNA expression. Skin was extirpated from the resulting F1 Tg mice (0 week old), and RNA was isolated from each skin. Expression of mRNA was evaluated by using the obtained RNA by a method similar to that of Example 3, using a Northern blot hybridization assay. Excessive expression of AGF mRNA was found in three strains of Tg mice.

The obtained three strains of the F1 Tg mice were mated with Balb/c mice to produce F2 mice. The mating with the Balb/c mice was repeated to produce F3 and F4 mice. The homozygotes were produced by mating the F4 Tg mice. These F3 and F4 mice were also assayed as in the case of the F1 mice to determine whether they were Tg mice or nontransgenic mice, by the PCR method using the primers comprising the SEQ ID NO: 10 and SEQ ID NO: 11. In the following Examples, the Tg mice used were the F3 or F4 Tg mice obtained as described above, and the normal mice used were the F3 or F4 nontransgenic mice from the same litter as the Tg mice.

### [Example 9] Histological staining of Tg mice

The Tg mice with excessive expression of AGF mRNA (hereinafter referred to as AGF Tg mice) exhibited appearance such as reddish skin, swelling, and curled body hair, which are different from the normal mice. To further investigate the effects on the skin, tissue sections were prepared for analysis (Noji, S. ed., Jikkenigaku (Experimental Medicine), Special Edition, The Protocol Series, Immunostaining and *in situ* Hybridization, Yodosha).

When the sections of the ear tissue fixed with 4% paraformaldehyde were stained with hematoxin-eosin, it was found that the epidermal cell layer of the AGF Tg mice was markedly thickened as compared to that of the normal mice. The epidermis of the ear of a normal mouse typically contained 1 to 2 cell layers, whereas, in the heterozygote AGF Tg mouse, it contained 3 to 5 layers of epidermal cells and in the homozygote, it contained 5 to 8 layers of epidermal cells, indicating that the degree of epidermal thickening increases with the amount of the AGF expressed. These phenotypes resembled the skin symptoms of patients suffering from psoriasis vulgaris.

### [Example 10] Enhancement of the proliferation activity of the epidermal cells in Tg mice

Next, the proliferation activity of the cells of the thickened epidermal tissue was examined in further detail. The BrdU Staining Kit (Oncogene Research Products) was used according to the manufacture's protocol. 100 µL of 10 mg/ml bromodeoxyuridine (BrdU) was administered to peritoneal cavity of a normal mouse and an AGF Tg mouse. The ear was excised and tissue sections were prepared one hour after the administration. While BrdU was little incorporated into the epidermal cells of the normal mouse, BrdU was incorporated into the AGF Tg mouse at very high levels. BrdU was incorporated in about 50% of the epidermal cells in the basal layer (FIG. 1, panels A and B, and graph E).

In addition, Phospho-Histone H3, which is specifically present in M-phase cells, was stained to examine the DNA synthesis capability of the cells. When the ear tissue sections prepared in a manner similar to that for the hematoxin-eosin staining were stained with an anti phoshohistone H3 antibody, epidermal cells of the normal mouse were scarcely stained while many epidermal cells of the basal layer in the AGF Tg mouse were stained (FIG. 1, panels C and D, and graph F).

These results indicate that the epidermal cells of the AGF Tg mouse display enhanced proliferation activity. These phenotypes were similar to the skin symptoms of patients suffering from psoriasis vulgaris.

### [Example 11] Angiogenesis and vascular permeability of Tg mice

AGF Tg mice were found to exhibit reddishness and swelling of skin with aging, in particular, near the eye, ear, nose, and mouth. Accordingly, angiogenesis in the skin tissue of the Tg mice was examined in detail. When the ear tissue sections of the AGF Tg mouse and the normal mouse were stained with hematoxin-eosin, a marked increase of microvessels was observed in the dermis of the AGF Tg mice, in particular, in the vicinity of the epidermal tissue, where AGF protein is expressed.

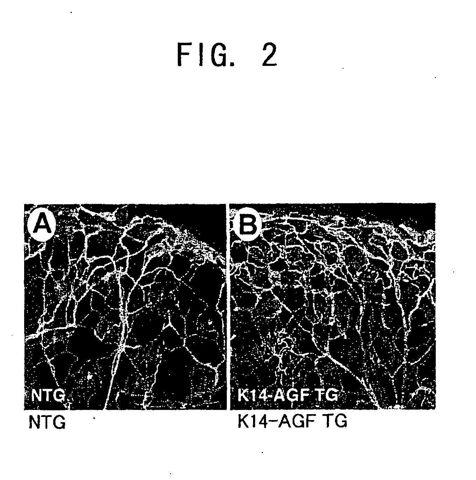

Next, blood vessels were visualized with FITC and observed. Fluorescein-labeled Lycopersicon esculentum lectin (Vector Laboratories) was administered to the tail vein of the AGF Tg mouse and the normal mouse under anesthesia. After five minutes, 1% paraformaldehyde and 0.5% glutaraldehyde were perfused from left ventricle for five minutes. The ear was excised, and the cartilage tissue was removed, thereby observing the blood vessels fluorescence-labeled by the staining with lectin under a fluorescence microscope. The results indicate that the amount of the blood vessel per unit area is increased in the AGF Tg mouse as compared to the normal mouse (FIG. 2).

Vascular permeability of the AGF Tg mouse was also examined. 100 µL of 1% Evans blue dye (Sigma)-PBS was administered to the tail vein of an anaesthetized mouse. The chest was opened 60 minutes after the administration for perfusion of 1% paraformaldehyde citrate buffer (pH 3.5) from left ventricle. The ear was excised, and the Evans blue dye was extracted with 1 ml formamide and absorption at 610 nm was measured with an absorption spectrophotometer to determine the amount of the Evans blue dye. The results indicate that the amount of the Evans blue dye is about three times higher in the AGF Tg mouse, and thus, the vascular permeability is markedly enhanced as compared to the normal mouse.

These results indicate that not only angiogenesis but also the vascular permeability is enhanced in the AGF Tg mouse.

### [Example 12] Isolation of mouse epidermal cells

Since significant proliferation of the epidermal cells in the AGF Tg mouse was observed in Example 10, the AGF protein was determined to have epidermal cell proliferating activity. Accordingly, in order to confirm whether the AGF directly acts on the epidermal cells, the epidermal cells of the mouse were isolated for examination of the proliferative activity of the AGF. C57BL/6 neonatal mice of 0 to 6 days after the birth were euthanized by placing them in ice, and sterilized with ethanol. The skin of the mouse was excised and put on 0.25% trypsin-EDTA solution (Lifetechnologies) in a 6 well dish at 4°C overnight. The epidermis and the dermis of the skin were then separated with forceps, and the epidermis was cut into small pieces with scissors, and the pieces were allowed to react in 0.05% collagenase solution for 2 hours. After separating the cells by passing through a 20G injection needle several times, the solution was passed through a 40 µm Nylon filter (Falcon) to obtain the epidermal cells. The epidermal cells thus obtained were stained with anti-β1 integrin antibody (Clone Ha2/5, PharMingen), and then, with FITC-labeled anti-mouse IgG antibody. The stained cells were separated into the fraction which was weakly positive to β1 integrin and the fraction which was strongly positive to β1 integrin by FACS Vantage (Becton Dickinson).

### [Example 13] Proliferative activity of OP9/AGF for mouse epidermal cells

The OP9 cell line with stable expression of AGF (OP9/AGF) and the control OP9 cell line (OP9/vector) produced in Example 4 were cultivated to confluence in a 12 well plate. To this plate, the mouse epidermal cells which were weakly positive and the mouse epidermal cells which were strongly positive to β1 integrin produced in Example 12 were inoculated at 4 x 10³ cells/well. The cells were cultivated in KGM-2 medium (Clonetics) with no addition of EGF for 7 days. The cells were then reacted in a solution containing 4% paraformaldehyde and 0.02% glutaraldehyde at 4°C for 10 minutes, and after the reaction, the cells were washed with PBS. The cells were further reacted in 40% methanol at 4°C for 5 minutes, in 80% methanol at 4°C for 5 minutes, in 100% methanol at 4°C for 10 minutes, in 80% methanol at 4°C for 5 minutes, and 40% methanol at 4°C for 5 minutes in this order for fixation of the cells. The cells were further reacted by using anti-keratin antibody as the primary antibody, and HRP-labeled anti-rabbit antibody as the secondary antibody, and the color was developed by using nickel chloride and DAB substrate to count the number of colonies comprising 10 to 50 cells and the number of colonies comprising 51 or more cells.

In the cells weakly positive to β1 integrin, the number of colonies comprising 10 to 50 cells was about 4 in the case of the OP9/vector whereas the number was about 13 in the OP9/AGF, and the number of colonies comprising 51 or more cells was about one in OP9/vector whereas the number was about 22 in the OP9/AGF. In the cells strongly positive to β1 integrin, the number of colonies comprising 10 to 50 cells was about 1 in the OP9/vector whereas the number was about 14 in the OP9/AGF, and the number of colonies comprising 51 or more cells was about 0 in the OP9/vector whereas the number was about 3 in the OP9/AGF. These results indicate that the AGF protein expressed by the OP9/AGF directly acts on the isolated epidermal cells to proliferate the epidermal cells.

### [Example 14] Wound healing effects

Since AGF has the activity of proliferating epidermal cells, it was predicted to be an effective promoter of the wound healing. Using the wound healing model (Lifei Guo *et al*., Genes & Dev. 10(2): 165-175 (1996); G. B. Mann *et al*., Cell 73(2): 249-261 (1993)), the activity of AGF in promoting wound healing was demonstrated. Ears were excised from the AGF Tg mice and the normal mice. Tissues, including the excised surface, were collected 1, 2, 3, 5, and 8 days after the excision to prepare the tissue sections. The tissue sections were stained with hematoxin-eosin, immunohistologically stained with an anti K14 antibody and with an anti AGF antibody to evaluate the wound healing process by observing the amount of the epidermal cells migrating from surrounding epidermis to the wound site and the amount of the proliferated epidermal cells. The first migration of the epidermal cells to the wound site was observed on the first day in the case of the AGF Tg mouse, while it was the second to third day in the normal mouse. The full coverage of the wound site with the epidermal cells was the second to the third day in the AGF Tg mouse while it was the third to the fifth day in the normal mouse. These results demonstrated the promotion of the wound healing by the AGF.

### [Example 15] Activity of closing the ear punch hole in AGF Tg mice

Since the AGF protein was demonstrated to have the activity of proliferating epidermal cells and healing wounds, action of the AGF protein on the dermal tissue, the subcutaneous tissue, and the cartilage tissue was also examined. A mouse ear is composed of epidermal tissue, dermal tissue, subcutaneous tissue, and cartilage tissue. A hole was punched in the ear of the mouse to examine the activity of the AGF protein to repair these lost tissues.

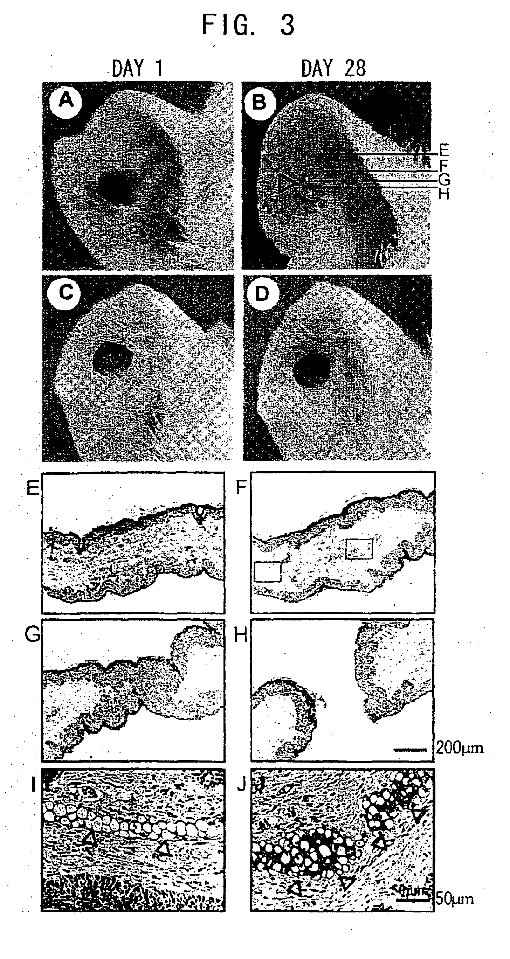

By penetrating through the center of the ear of 8 week old AGF Tg mice and normal mice, a hole with a diameter of 2 mm (hereinafter referred to as the ear punch hole) was formed by using a metal ear punch (Natsume Seisakusho). The mice were raised under normal conditions, and size of the ear punch hole was observed. While the ear punch hole of the normal mice showed substantially no change in its size (FIG. 3, C and D), the ear punch hole of the AGF Tg mice became smaller over time, becoming almost fully closed on the 28th day after the punching of the hole (FIG. 3, A and B). It was thus demonstrated that the AGF protein has the activity of regenerating ear tissue and closing the hole, since the AGF protein is expressed from epidermal cells of the ear in the AGF Tg mouse.

Next, the regenerated ear tissue was observed. The ear was excised from the AGF Tg mouse raised for 28 days after the hole punching, and the tissue was fixed in 4% paraformaldehyde, embedded in paraffin and sliced into thin sections. As shown in FIG. 3 B, thin sections were prepared from the region (E) with no ear punching and the regions (F, G, and H) where holes had been punched and the tissue had then regenerated. Since the tissue regenerates from the peripheral region to the center, with the hole becoming smaller over time, the regions F, G, and H in FIG. 3 B regenerate in the order of F, G, and H. The thin sections were stained with hematoxin-eosin, observed under a microscope and photographed (FIG. 3, E to H). FIG. 3 F includes the region (within the frame on the left) from the tissue without the ear punch hole and the region (within the frame in the center) where the hole had been punched and the tissue had then regenerated, and the photographs were taken by magnifying these regions (FIG. 3, I and J). In FIG. 3 I and J, some parts of the cartilage tissue are shown by the arrowheads.

Tissue of a mouse ear typically comprises a laminar structure comprising, from the outermost layer, the epidermal, dermal, and subcutaneous tissues, and the cartilage tissue in the center (FIG. 3, E). In the region of the tissue regeneration, the epidermal and dermal tissues were formed first, and subsequently, the subcutaneous tissue was formed with blood vessels. Formation of the cartilage tissue in the center of the subcutaneous tissue was then observed (FIG. 3, F and J).

The ear punch hole is used for identification of individual mice since the ear punch holes are generally never closed. No factor having the activity of closing the mouse ear punch hole has been known to date. A similar phenomenon is only found in MRL mice, but the factor causing such closure of the ear punch hole in the MRL mice has not yet been identified (Clark L.D. *et al*., Clin Immunol Immunopathol 1998, 88(1), 35-45). The AGF protein was found to have very strong tissue regenerative activity that had previously been unknown. The AGF protein was also found to have the activity of regenerating dermal tissue, subcutaneous tissue, and cartilage tissue, in addition to epidermal tissue. Since tissue regeneration requires proliferation of cells constituting the tissue, the AGF protein was found to have the activity of proliferating the cells constituting such tissue, namely, fibroblasts, endothelial cells, chondrocytes, etc. Because of such activity of regenerating the cartilage tissue, AGF protein was estimated to be useful as a therapeutic agent for osteoarthritis.

### [Example 16] Activity of closing the ear punch hole of the recombinant AGF from OP9 cells

The activity of closing the ear punch hole by AGF was evaluated using the OP9 cell line expressing recombinant AGF. A punch hole with the diameter of 2 mm was formed in the ear of an 8 week old Balb/c nude mouse, and a picture was taken after 1 hour (FIG. 4). The cell line with stable expression of mouse AGF (OP9/AGF) and the OP9 cell line (OP9/vector) transfected only with pEF-BOS-neo vector and free from the mouse AGF gene produced in Example 4 were cultivated. Before administration to the mouse, the cells were detached from the culture plate by treating with trypsin and suspended in PBS. On the 4th, 8th, 12th, and 20th day of the ear hole punching, 5 x 10⁶ cells of the OP9/AGF or the OP9/vector suspended in PBS were administered to peritoneal cavity of the Balb/c nude mouse. As a result, while the ear punch hole of the OP9/vector-administered mouse showed no change in its size, the ear punch hole of the OP9/AGF-administered mouse became smaller over time and the ear punch hole became remarkably small on the 20th day. The OP9/AGF-administered mouse and the OP9/vector-administered mouse were photographed on the 20th day of the ear hole punching (FIG. 4).

OP9/AGF has been found to express recombinant AGF. Accordingly, it was found that, in the mouse having OP9/AGF administered to its peritoneal cavity, the recombinant AGF expressed by the OP9/AGF repaired the tissue that had been lost by the ear hole punching to reduce the size of the hole.

### [Example 17] Activity of AGF for proliferating cartilage cells

Activity of AGF for proliferating the cartilage cells was examined *in vitro.* pEGFPMY (Onai N. *et al*., Blood, 96(6), 2074-2080, 2000), which is an expression vector for green fluorescent protein (GFP) that can be used in retroviral expression systems, was introduced into the mouse cartilage precursor cell line, ATDC5. Production of the retrovirus and infection of ATDC5 were carried out by the method of Miyamoto *et al*. (Miyamoto T. *et al*., Blood, 98(8), 2544-2554, 2001).

The infected cell was cultivated in ATDC5 culture medium (DMEM/F-12 (Lifetechnologies), 5% FCS, 5 micrograms/ml insulin, 5 micrograms/ml transferrin, and 3 x 10⁻⁸ mol/liter sodium selenite). Before reaching confluence, the cells were detached from the culture plate by treating with trypsin for cell dispersion. The cells thus dispersed were applied to a cell sorter (FACS vantage, Becton Dickinson) to separate and collect the ATDC5 with the GFP fluorescence to obtain the ATDC5 expressing the GFP. The resulting GFP-expressing ATDC5 was cultivated and proliferated, and the cells were applied again to the cell sorter for separation and collection of the GFP-expressing ATDC5 as described above to obtain the ATDC5 stably expressing the GFP (ATDC5/GFP). The OP9 cell line with the stable expression of AGF (OP9/AGF) and the control OP9 cell line (OP9/vector) produced in Example 4 were cultivated to confluence in a 12 well plate. ATDC5/GFP was inoculated in this plate at 50 cells/well, and cultivated in ATDC5 culture medium for 14 days. Proliferation of the ATDC5/GFP was examined under a fluorescence microscope.

It was then found that, when the OP9/vector was used as the feeder cells, no ATDC5/GFP colony was formed and ATDC5/GFP did not proliferate. On the other hand, when OP9/AGF was used as the feeder cells, many ATDC5/GFP colonies were formed (about 16 colonies per well) and the ATDC5/GFP markedly proliferated. Since OP9/AGF expresses the recombinant AGF protein, it was demonstrated that recombinant AGF has the activity of proliferating the ATDC5/GFP cells, namely, that recombinant AGF had the activity of proliferating the cartilage cells.

### [Example 18] Cloning of human AGF gene

For the amplification of the human AGF gene, the forward primer used was 5'-ATGGGGAAGCCCTGGCTGCGTGCGCTACAG-3' (SEQ ID NO: 12) and the reverse primer used was 5'-TCACAGCTTCAGGGGCCGAATGAGCATGGC-3' (SEQ ID NO: 13). PCR was conducted by using PyroBest™ DNA polymerase (Takara Shuzo) in the presence of 5% formamide, repeating 35 times the cycle of 98°C for 20 seconds, 64°C for 30 seconds, and 74°C for 3 minutes. As a consequence, a DNA fragment of about 1.5 kbp was amplified. This fragment was cloned by using pCR2.1 plasmid (Invitrogen). The nucleotide sequence of the resulting clone was analyzed by dideoxy termination method using ABI377 DNA Sequencer (Applied Biosystems), and the fragment was confirmed to be the sequence defined in SEQ ID NO: 1.

### [Example 19] Expression and purification of mouse AGF and human AGF

PCR was conducted using a plasmid comprising the full length cDNA as the template, and PyroBest™ DNA polymerase (Takara Shuzo), under conditions of 94°C for 2 minutes, and then 15 cycles of 98°C for 20 seconds, 55°C for 30 seconds, and 72°C for 1 minute 30 seconds, followed by 72°C for 7 minutes. The primer sets used were: CGGGATCCACCGGGCGGGCGCCCCGCGCTGC (SEQ ID NO: 14) and CGCTCGAGTCACAGCTTCAGGGGCCGAATGAG (SEQ ID NO: 15) for the human AGF, and CGGGATCCACAGGGCGCTCGGAGGTGCCGCG (SEQ ID NO: 16) and CGCTCGAGTCACAAGCGCACAAGCCGGGTCAA (SEQ ID NO: 17) for the mouse AGF. The fragment thus formed was subcloned into the EcoRV site of pZEr02.1 (Invitrogen), and then cleaved with BamHI and XhoI. The resulting DNA fragment of about 1.4 kbp (human) or about 1.3 kbp (mouse) was inserted into the BamHI-XhoI site of pcDNA-Signal-FLAG to complete the expression plasmid. The expression plasmid was then introduced into HEK293 cells using a transfection reagent (FuGENE™6 Transfection Reagent; Roche) according to the manufacturer's instruction. Two days after plasmid introduction, the culture medium was replaced with a medium containing 200 µg/ml of G418, and the cultivation was continued for another 5 days. The thus obtained G418 resistant cells were diluted to 1 cell/well, inoculated in a 96 well plate, and the cultivation was continued. By observing the cells under a microscope, clones forming a single colony with high growing speed were selected, and the culture supernatants of these clones were analyzed by Western blotting to obtain the cell clones with high expression. The human AGF exhibited a protein expression of about 2 µg/ml in clone hNEW #2 and the mouse AGF exhibited a protein expression of about 0.1 µg/ml in clone mNEW #1.

pcDNA3.1-signal-FLAG is a plasmid in which a double stranded oligo-DNA comprising SEQ ID NO: 18 and SEQ ID NO: 19 has been inserted at HindIII-XhoI site of pcDNA3.1(+) (Invitrogen) by the method described in WO 01/34785 (Example 7-1).

The cell lines (mNEW #1 and hNEW #2) expressing the mouse AGF and the human AGF, respectively, were cultivated to obtain the culture supernatant. The culture supernatant was applied to ANTI-FLAG M2 Monoclonal Antibody Agarose Affinity Gel (Sigma), washed with phosphate buffered saline (PBS), and eluted with 10 mmol/L-Tris-HCl (pH 3.0). The eluate was dialyzed against PBS to obtain purified protein. The purified protein was electrophoresed under reducing conditions, and the protein was stained with SYPRO™ Orange Protein Gel Stain (BIO-RAD). As a consequence, the mouse AGF was purified as a protein having a molecular weight of about 55 kDa and the human AGF was purified as a protein having a molecular weight of about 60 kDa.

### [Example 20] Activity of proliferating epidermal cells

C57BL/6 neonatal mice of 0 to 2 days after birth were euthanized by placing in ice, and sterilized with ethanol. The skin of the mouse was excised and put on 0.25% trypsin-EDTA solution (Lifetechnologies) in a 6 well dish at 4°C overnight. The epidermis and the dermis of the skin were then separated with forceps, and the epidermis was cut into small pieces with scissors. The pieces were allowed to react in 0.05% collagenase solution for 2 hours. After separating the cells by passing through a 20G injection needle several times, the solution was passed through a 40 µm Nylon filter (Falcon) to obtain the epidermal cells. The thus obtained epidermal cells were cultivated in KGM-2 medium (Clonetics) for one day, and the cells which adhered were detached by treating with trypsin for use in the following assay.

OP9 cells were cultivated to confluence in a 12 well plate. To this plate, mouse epidermal cells were inoculated at 1000 cells/well with or without 9 µg/ml of the purified mouse AGF, and cultured in an EGF-free KGM-2 medium for 5 days. After 5 days of cultivation, the cells were reacted in a solution containing 4% paraformaldehyde and 0.02% glutaraldehyde at 4°C for 10 minutes, washed in PBS, and further reacted in 40% methanol at 4°C for 5 minutes, in 80% methanol at 4°C for 5 minutes, in 100% methanol at 4°C for 10 minutes, in 80% methanol at 4°C for 5 minutes, and in 40% methanol at 4°C for 5 minutes in this order for fixation of the cells. The cells were further reacted by using an anti-keratin antibody as the primary antibody and an HRP-labeled anti-rabbit antibody as the secondary antibody. The color was developed using the DAB substrate, and the number of colonies comprising 10 or more cells was counted.

It was then found that the number of the epidermal cell colonies was about 14/well with no addition of the mouse AGF whereas the number of the epidermal cell colonies was about 28/well when the mouse AGF was added. This demonstrates the ability of the purified AGF protein to induce the epidermal cells to proliferate.

### Industrial Applicability

The present invention provides polypeptides having epidermal cell proliferating activity. The polypeptides of the present invention can be used for treating wounds by utilizing its action of proliferating the epidermal cells. Effectiveness of angiopoietin-related growth factors (AGFs) in the wound healing has been demonstrated in Examples 13 and 14. The polypeptides of the present invention also produce phenotypes resembling the skin conditions of the patients suffering from psoriasis by the manipulated expression of the polypeptides in the epidermal cells. Therefore, the polypeptides of the present invention are useful for producing an animal model for psoriasis.

The present invention has also clarified the action of the mouse AGF. In addition, the present invention has revealed that human NL8/NEW is an ortholog of mouse AGF, namely, that NL8/NEW is the human AGF. The human AGF has 76% homology to the mouse AGF at the amino acid level, and in particular, a homology of as high as 89% in the fibrinogen domain on the C terminal side, a domain known to be important in the activity of 'the proteins belonging to angiopoietin family (William N. Procopio *et al*., J. Biol. Chem. 274(42): 30196-30201 (1999)). Such high homology within the domain postulated to play a significant role in the maintenance of physiological activity suggests that human AGF has an activity equivalent to that of mouse AGF. Therefore, human AGF and its gene, like mouse AGF, are expected to be useful in wound healing. Accordingly, the present invention has demonstrated the utility of human AGF and the AGF gene in the wound healing.

The present invention also provides Tg animals expressing AGF. The Tg mouse manipulated to express AGF in the epidermal cells exhibited an enhanced vascular permeability (Example 11). Therefore, the Tg animals of the present invention are useful as model animals of allergic diseases, urticaria, edema, and inflammation. In addition, since the Tg animals of the present invention exhibited increased angiogenesis (Example 11), they are also useful as model animals of solid cancer, RA, and diabetic retinopathy. Furthermore, since the Tg animals of the present invention exhibited thickening of the epidermis (Example 9) as well as enhanced cell proliferative activity of the epidermal cells (Example 10), they are useful as model animals of psoriasis. As described above, the present invention provides Tg animals useful as model animals of allergic diseases, urticaria, edema, inflammation, solid cancer, RA, diabetic retinopathy, and psoriasis.

Furthermore, the AGF polypeptides/polynucleotides of the present invention have activity of proliferating fibroblasts, endothelial cells, chondrocytes, and the like, and the activity regenerating not only epidermal tissue but also dermal tissue, subcutaneous tissue, and cartilage tissue. In other words, the AGF polypeptides/polynucleotides of the present invention are effective in tissue regeneration. Regeneration of cartilage tissue has proven to be difficult. Nevertheless, the tissue regenerative agents of the present invention have been shown to have a regenerative action on such cartilage tissue. In other words, the tissue regenerative agents of the present invention have regenerative action on the tissues whose regeneration is impossible by conventional techniques. As described above, the present invention greatly contributes to the field of tissue regeneration medicine.

## Claims

1. A polypeptide of any one of the following (1) to (4):
(1) a polypeptide comprising the amino acid sequence of 1 to 433 of SEQ ID NO: 4, wherein said polypeptide has epidermal cell proliferating activity;
(2) a polypeptide consisting of the amino acid sequence of 1 to 433 of SEQ ID NO: 4, wherein substitution, deletion, and/or insertion of one to several amino acid residues has occurred at one to several sites of the amino acid sequence, wherein said polypeptide has epidermal cell proliferating activity;
(3) a polypeptide encoded by a DNA which hybridizes with the polynucleotide shown in the nucleotide sequence of 73 to 1371 of SEQ ID NO: 3 under stringent conditions, wherein said polypeptide has epidermal cell proliferating activity; and
(4) a polypeptide consisting of an amino acid sequence having a homology of at least 95% to the amino acid sequence of 1 to 433 of SEQ ID NO: 4, wherein said polypeptide has epidermal cell proliferating activity.

2. A polypeptide of one of the following (1) or (2):
(1) a polypeptide consisting of the amino acid sequence of 1 to 433 of SEQ ID NO: 4; and
(2) a polypeptide consisting of the amino acid sequence of SEQ ID NO: 4.

3. A polynucleotide encoding the polypeptide of claim 1 or 2.

4. A vector comprising the polynucleotide of claim 3 in an expressible form.

5. A host cell comprising the vector of claim 4.

6. A non-human transgenic animal manipulated to express the polypeptide of claim 1 or 2 in its epidermis.

7. An animal model for psoriasis comprising a non-human transgenic animal manipulated to express in its epidermis a polypeptide of any one of the following (a) to (d):
(a) a polypeptide comprising the amino acid sequence of 1 to 450 of SEQ ID NO: 2 or the amino acid sequence of 1 to 433 of SEQ ID NO: 4, wherein said polypeptide has epidermal cell proliferating activity;
(b) a polypeptide comprising the amino acid sequence of 1 to 450 of SEQ ID NO: 2 or the amino acid sequence of 1 to 433 of SEQ ID NO: 4, wherein substitution, deletion, and/or insertion of one to several amino acid residues has occurred at one to several sites of said amino acid sequence, wherein said polypeptide has epidermal cell proliferating activity;
(c) a polypeptide encoded by a DNA which hybridizes with the DNA encoding the amino acid sequence of 1 to 450 of SEQ ID NO: 2 or the amino acid sequence of 1 to 433 of SEQ ID NO: 4 under stringent conditions, wherein said polypeptide has epidermal cell proliferating activity; and
(d) a polypeptide consisting of an amino acid sequence having a homology of at least 95% to the amino acid sequence of 1 to 450 of SEQ ID NO: 2 or to the amino acid sequence of 1 to 433 of SEQ ID NO: 4, wherein said polypeptide has epidermal cell proliferating activity.

8. A tissue regenerative agent comprising as its active ingredient a polypeptide of any one of the following (a) to (d) :
(a) a polypeptide comprising the amino acid sequence of 1 to 450 of SEQ ID NO: 2 or the amino acid sequence of 1 to 433 of SEQ ID NO: 4, wherein said polypeptide has epidermal cell proliferating activity;
(b) a polypeptide comprising the amino acid sequence of 1 to 450 of SEQ ID NO: 2 or the amino acid sequence of 1 to 433 of SEQ ID NO: 4, wherein substitution, deletion, and/or insertion of one to several amino acid residues has occurred at one to several sites of said amino acid sequence, wherein said polypeptide has epidermal cell proliferating activity;
(c) a polypeptide encoded by a DNA which hybridizes with the DNA encoding the amino acid sequence of 1 to 450 of SEQ ID NO: 2 or the amino acid sequence of 1 to 433 of SEQ ID NO: 4 under stringent conditions, wherein said polypeptide has epidermal cell proliferating activity; and
(d) a polypeptide consisting of an amino acid sequence having a homology of at least 95% to the amino acid sequence of 1 to 450 of SEQ ID NO: 2 or to the amino acid sequence of 1 to 433 of SEQ ID NO: 4, wherein said polypeptide has epidermal cell proliferating activity.

9. The tissue regenerative agent according to claim 8, wherein the tissue is selected from the group consisting of skin tissue, epithelial tissue, cartilage tissue, and connective tissue.

10. The tissue regenerative agent according to claim 8 or 9, wherein the tissue regeneration is wound healing.

11. The tissue regenerative agent according to claim 8 or 9, wherein the tissue regenerative agent is a therapeutic agent for osteoarthritis.

12. A tissue regenerative agent comprising as its active ingredient a polynucleotide of any one of the following (e) to (h) :
(e) a polynucleotide comprising the nucleotide sequence of 61 to 1410 of SEQ ID NO: 1 or the nucleotide sequence of 73 to 1371 of SEQ ID NO: 3, wherein said polynucleotide encodes a polypeptide having epidermal cell proliferating activity;
(f) a polynucleotide encoding a polypeptide comprising the amino acid sequence of 1 to 450 of SEQ ID NO: 2 or the amino acid sequence of 1 to 433 of SEQ ID NO: 4, wherein substitution, deletion, and/or insertion of one to several amino acid residues has occurred at one to several sites of said amino acid sequence, wherein said polypeptide has epidermal cell proliferating activity;
(g) a polynucleotide which hybridizes with the DNA shown in the nucleotide sequence of 61 to 1410 of SEQ ID NO: 1 or the nucleotide sequence of 73 to 1371 of SEQ ID NO: 3 under stringent conditions, wherein said polynucleotide encodes a polypeptide having epidermal cell proliferating activity; and
(h) a polynucleotide encoding a polypeptide consisting of an amino acid sequence having a homology of at least 95% to the amino acid sequence of 1 to 450 of SEQ ID NO: 2 or to the amino acid sequence of 1 to 433 of SEQ ID NO: 4, wherein said polypeptide has epidermal cell proliferating activity.

13. The tissue regenerative agent according to claim 12, wherein the tissue is selected from the group consisting of skin tissue, epithelial tissue, cartilage tissue, and connective tissue.

14. The tissue regenerative agent according to claim 12 or 13, wherein the tissue regeneration is wound healing.

15. The tissue regenerative agent according to claim 12 or 13, wherein the tissue regenerative agent is a therapeutic agent for osteoarthritis.
